# EUROPEAN PATENT APPLICATION

(11) **EP 4 616 801 A1**
(43) Date of publication of application: **17.09.2025**
(21) Application number: 22964685.6
(22) Date of filing: 07.11.2022
(51) Int. Cl.: A61B 5/15

(54) **APPARATUS AND METHOD FOR COLLECTING BLOOD OR OTHER LIQUIDS OF SUBJECT**

(71) Applicant: MGI Tech Co., Ltd., Shenzhen, Guangdong 518083 (CN)
(72) Inventor: GU, Xianyong, Shenzhen, Guangdong 518083 (CN); YANG, Meng, Shenzhen, Guangdong 518083 (CN); BARTMAN, Jon, Shenzhen, Guangdong 518083 (CN); LI, Lei, Shenzhen, Guangdong 518083 (CN); LIU, Fangwei, Shenzhen, Guangdong 518083 (CN); DU, Bing, Shenzhen, Guangdong 518083 (CN); YAO, Fei, Shenzhen, Guangdong 518083 (CN)
(74) Representative: Wu, Ting
(86) International application number: PCT/CN2022/130371
(87) International publication number: WO 2024/098207

(57) **Abstract**

An apparatus for collecting blood or other liquids, comprising a housing (100), a starting device (200), a transmission device (300) and a collection device (400), wherein: the collection device (400) is housed in the housing (100); the transmission device (300) is housed in the housing (100), the transmission device (300) comprises a locking assembly (310) and a driving assembly (320), the driving assembly (320) is connected to the collection device (400), at least part of the starting device (200) is detachably connected to the locking assembly (310), and the locking assembly (310) is configured, to release a movement restriction on the driving assembly (320) after the starting device (200) is inserted, so that the driving assembly (320) actuates the collection device (400) to extend out of the housing (100), and to restrict a movement of the driving assembly (320) after the starting device (200) is removed. The apparatus effectively solves the problem of blood collection apparatus in the prior art that are prone to injury to a human body caused by extension of blood collection components due to accidental activation or contact, and the internal structure layout of the apparatus is more reasonable.

## Description

### TECHNICAL FIELD

This application relates to the field of medical devices, particularly to an apparatus and method for collecting blood or other liquids from a subject.

### BACKGROUND

At present, blood sampling from a human body primarily requires the assistance of professional medical personals. With the development of POCT (point of care testing) technology and changes in the testing mode in hospitals in the future, there will be an increasing number of situations where people need to complete blood collection on their own.

However, it is difficult for ordinary personnel without professional skills to complete blood collection alone, or to collect blood stably and quickly. There is an apparatus in the existing technology that uses a pressing structure similar to a ballpoint pen for self-service blood collection, wherein the principle is to cause a metal dome to deform by pressing and thus generate potential energy, then release the potential energy to drive a blade to quickly move and pierce into a skin, and finally push the blade back quickly by a spring at bottom, so as to complete blood collection.

However, the above-mentioned apparatus and apparatuses in related technologies are generally button based designs, which drive a blade or blood collection needle to extend and puncture a skin for blood collection by pressing a button, and it is easy to accidentally activate or contact the button during use, resulting in unnecessary damage.

In view of this, this application is hereby put forward.

### SUMMARY

This application provides an apparatus and method for collecting blood or other liquids from a subject, so as to solve the problem of blood collection apparatuses in the prior art that are prone to injury to a human body due to accidental activation or contact.

To solve the above problem, this application adopts the following solution:
This application provides an apparatus for collecting blood or other liquids in a first aspect, comprising a housing, a starting device, a transmission device, and a collection device;
The transmission device is housed in the housing, the transmission device comprises a locking assembly and a driving assembly, the driving assembly is connected to the collection device, at least part of the starting device is detachably connected to the locking assembly, and the locking assembly is configured to restrict a movement of the driving assembly before the starting device is inserted, and to release a movement restriction on the driving assembly after the starting device is inserted, so that the driving assembly actuates the collection device to extend out of the housing.

The apparatus for collecting blood or other liquids in this solution comprises a housing, a starting device, a transmission device and a collection device, the transmission device comprises a locking assembly and a driving assembly, the driving assembly is connected to the collection device and is configured to drive the collection device to extend and retract, the transmission device and the collection device are both provided inside the housing, the starting device is independent of the housing and is detachably connected to the locking assembly, and after the starting device is inserted, the locking assembly releases a movement restriction on the driving assembly, so that the driving assembly actuates the collection device to extend out of the housing, and after the starting device is removed, the locking assembly restricts a movement of the driving assembly.

In this solution, the starting device is detachably connected to the locking assembly. The collection device can only extend when the starting device is inserted into the locking assembly, which will not cause the collection device to extend due to accidental contact or activation, thereby effectively solving the problem of blood collection apparatuses in the prior art that are prone to injury to a human body caused by extension of blood collection components due to accidental activation or contact.

In other preferred solutions, the locking assembly comprises a pushing block and sliding pieces, the pushing block can move relative to the sliding pieces, the pushing block is configured to move along a first direction under an action of the starting device and drive the sliding pieces, and the sliding pieces are configured to move along a second direction under an action of the pushing block, for restricting a movement of the collection device in the first direction and releasing a movement restriction on the collection device.

When the starting device is inserted, the pushing block will drive the sliding pieces to move along the second direction, thereby restricting the movement of the collection device in the first direction and releasing the movement restriction on the collection device. In this solution, the first direction is not collinear with the second direction, which can make the overall layout of the apparatus more reasonable and avoid excessive elongation of the overall apparatus.

In other preferred solutions, the sliding piece comprises a first slider and a first deformable piece located between the first slider and an inner wall of the housing, the first deformable piece is connected to the first slider for reciprocating the first slider along the second direction. When the starting device is inserted, it will actuate the first slider to squeeze and compress the first deformable piece, and when the starting device is removed, the first deformable piece will return to its original state and actuate the first slider to reset.

In other preferred solutions, the sliding pieces are symmetrically arranged on both sides of the collection device along the second direction, and the pushing block is arranged between the two sliding pieces and pushes the two sliding pieces to move in opposite directions, this layout is more reasonable, and the pushing block actuates the sliding pieces to move synchronously toward opposite sides, which is more stable than single-side movement.

In other preferred solutions, the first slider has a body part and a position limiting part, the body part is connected to the first deformable piece, the position limiting part protrudes from the body part along the second direction, and the position limiting part is configured to at least partially stop the collection device in the first direction after the starting device is removed, and release a stop on the collection device after the starting device is inserted. The position limiting effect on the collection device is achieved through the position limiting part. Specifically, when the starting device is not inserted, the collection device is in a retracted state, and when the starting device is inserted, the position limiting part releases the position limiting effect on the collection device, allowing the collection device to extend.

In other preferred solutions, the pushing block has an inserting-connection part and squeezing parts, the inserting-connection part is configured to form an inserting connection with the starting device, and the squeezing parts protrude from an outer side of the inserting-connection part for squeezing the sliding pieces in the second direction.

In other preferred solutions, a cross-sectional area of the squeezing part gradually decreases along an insertion direction of the starting device, thereby facilitating the insertion of the starting device.

In other preferred solutions, the housing is provided with a guiding column;
the inserting-connection part comprises an inserting-connection section and a guiding section, the inserting-connection section is provided with an inserting-connection groove, the guiding section is sleeved on the guiding column, and the squeezing part is provided on the guiding section. When the starting device is inserted, the guiding section squeezes the sliding pieces in the second direction, causing the position limiting part to release its position limiting effect on the collection device, thereby allowing the collection device to extend.

In other preferred solutions, the sliding piece is provided with a positioning portion corresponding to the squeezing parts, and the positioning portion is provided with accommodation parts that can accommodate the squeezing parts, which are configured to limit the movement of the squeezing parts.

In other preferred solutions, the driving assembly is located between the collection device and an inner wall of the housing, the driving assembly comprises a driving shaft connected to the collection device and a driving elastic piece sleeved outside the driving shaft, and after the sliding pieces release the movement restriction on the collection device, the driving elastic piece pushes the driving shaft along the first direction and actuates the collection device to move towards outside of the housing to a collection position.

In other preferred solutions, the driving assembly further comprises a reset mechanism, which pushes the collection device to move towards inside of the housing after the collection device moves to the collection position, and the reset mechanism is separated from the driving elastic piece, so as to reset the collection device.

In other preferred solutions, the reset mechanism comprises a shaft sleeve sleeved outside the driving shaft and a reset elastic piece connected to the shaft sleeve, and the driving shaft is configured to pass through the shaft sleeve along the second direction and has a protrusion protruding outward; after the collection device moves to the collection position, the reset elastic piece pushes the shaft sleeve and actuates the driving shaft and the collection device to move towards inside of the housing through the protrusion.

In other preferred solutions, an end of the reset elastic piece away from the collection device is fixed to the shaft sleeve, and an end of the reset elastic piece close to the collection device is configured to be movable relative to the shaft sleeve along the second direction relative to the housing.

In other preferred solutions, both the driving elastic piece and the reset elastic piece are precompressed springs, the reset elastic piece is sleeved on an outer circumference of the shaft sleeve, and an elastic coefficient of the driving elastic piece is greater than that of the reset elastic piece.

In other preferred solutions, the driving assembly further comprises a top position-limiting mechanism located at an end of the driving elastic piece away from the collection device, and the top position-limiting mechanism is configured to precompress the driving elastic piece and the reset elastic piece.

In other preferred solutions, the top position-limiting mechanism comprises second sliders located on opposite sides of the driving shaft and position-limiting elastic pieces for actuating two second sliders to move relative to each other, at least two second sliders enclose an unobstructed part, and an end of the driving shaft away from the collection device extends outside the top position-limiting mechanism through the unobstructed part and is spaced apart from the top position-limiting mechanism.

In other preferred solutions, the top position-limiting mechanism includes second sliders located on opposite sides of the driving shaft and spaced apart from each other, as well as precompressed position-limiting elastic pieces connected between the two second sliders that are arranged to be opposite; the top sliders and the precompressed position-limiting elastic pieces enclose the unobstructed part; a position-limiting groove is provided on a side of the second slider close to the collection device, and the position-limiting groove is configured to form a snapping connection with the shaft sleeve;
when the collection device moves to the collection position, the driving elastic piece drives the driving shaft to actuate the shaft sleeve to move and disengage from the position-limiting groove, the position-limiting elastic piece drives the two second sliders to separate, the end of the driving elastic piece away from the collection device is released through a gap generated by a separation between the second sliders, and the reset elastic piece pushes the shaft sleeve and actuates the driving shaft and the collection device to move towards inside of the housing through the protrusion.

In other preferred solutions, the top position-limiting mechanism includes second sliders located on opposite sides of the driving shaft and pre-stretched position-limiting elastic pieces connected between the slider and the housing, and the second sliders are spaced to form the unobstructed part therebetween.

In other preferred solutions, the first direction is perpendicular to the second direction.

In other preferred solutions, the housing is provided with an installation port and a collection port, the starting device is detachably connected to the locking assembly via the installation port, and the transmission device drives the collection device to extend out of the housing through the collection port along the second direction.

In other preferred solutions, a first fluid channel which is in communication with the collection port is housed in the housing, and the starting device is in communication with the collection port through the first fluid channel.

In other preferred solutions, the starting device comprises a starting assembly and a first puncturing assembly, the starting assembly is detachably connected to the locking assembly, the first puncturing assembly is detachably connected to the starting assembly, and the first puncturing assembly is configured to pierce the starting assembly when the starting assembly is inserted into the locking assembly of the transmission device, so that the starting assembly is in communication with the collection device via the first puncturing assembly.

In other preferred solutions, the starting assembly comprises a connector, a storage tube extending from the connector, and a negative pressure tube in communication with the storage tube, the connector is configured to be detachably connected to the locking assembly, the negative pressure tube has a pressure lower than atmospheric pressure, and the storage tube is configured to be in communication with the collection device through the first puncturing assembly when the connector is inserted into the locking assembly, for storing the blood or other liquids collected by the collection device.

In other preferred solutions, the collection device includes a housing with an accommodation space and a puncturing assembly which is at least partially accommodated within the accommodation space; the housing includes a contact wall, and the collection port penetrates the contact wall; the puncturing assembly is movable relative to the contact wall and is configured to pierce the barrier through the collection port;
the contact wall includes a contact face close to the barrier; the contact face is recessed in the direction away from the barrier to form a recess; the recess includes a bottom wall opposite to the barrier and a surrounding wall which extends from the bottom wall towards the barrier and is connected between the contact face and the bottom wall;
the bottom wall is provided with a central part which protrudes towards the barrier and is spaced apart from the surrounding wall, the collection port is provided around an outer circumference of the central part and located between the surrounding wall and the central part.

In other preferred solutions, the puncturing assembly comprises at least one microneedle, and the bottom wall is perforated with a through hole(s), which corresponds to the at least one microneedle one by one, opposite to the collection port.

In other preferred solutions, an end of the second fluid channel away from the negative pressure tube extends into the accommodation space and is in communication with the collection ports.

In other preferred solutions, the contact wall includes a first contact wall and a second contact wall which are stacked along the movement direction of the puncturing assembly; the contact face is located on the side of the first contact wall away from the second contact wall, and the first contact wall is penetrated to form the surrounding wall; the bottom wall is located on the side of the second contact wall close to the first contact wall, and the central part is formed by extending from the second contact wall towards the first contact wall.

In other preferred solutions, the second fluid channel is provided on the second contact wall and the end away from the central part penetrates the second contact wall and extends into the housing.

In other preferred solutions, the end face of the central part away from the bottom wall is coplanar with the contact face.

In other preferred solutions, the collection device further comprises a flexible seal which is configured to cover the side of the contact wall facing away from the contact face and connected between the puncturing assembly and the contact wall;
the flexible seal includes a central fixed part fixed to the puncturing assembly, an edge fixed part surrounding the central fixed part, and a flexible sealing ring connected between the central fixed part and the edge fixed part; the puncturing assembly extends through the central fixed part towards the contact wall, and the orthogonal projection of the flexible seal onto the contact wall covers the whole collection port.

In other preferred solutions, the flexible sealing ring and the central fixed part move with the puncturing assembly.

In other preferred solutions, the puncturing assembly further comprises a needle holder associated with the transmission device, and the microneedle is fixed to the needle holder.

In other preferred solutions, the starting device also includes a connector that is detachably connected to the locking assembly, and a storage tube that extends from the connector; the negative pressure tube is in communication with the storage tube, and the storage tube is located between the connector and the negative pressure tube; the storage tube is in communication with the second fluid channel through the connector.

In other preferred solutions, the housing has a mounting part, and one of the mounting part and the connector includes a first piercing piece which has a hollow tubular shape with two end installation ports, and the other one includes a pierceable seal corresponding to the first piercing piece; the first piercing piece is in communication with the second fluid channel; when the mounting part is connected to the connector, the first piercing piece pierces the pierceable seal and establishes communication between the storage tube and the second fluid channel.

In other preferred solutions, the first piercing piece is connected to the mounting part; the locking assembly is provided at the mounting part, and when the starting device is inserted into the locking assembly, the first piercing piece pierces the pierceable seal and is in communication with the negative pressure tube.

In other preferred solutions, the starting device further comprises a capillary tube connected to the storage tube, and the storage tube is in communication with the negative pressure tube through the capillary tube.

In other preferred solutions, the storage tube comprises a first open end connected to the connector, and a second open end opposite to the first open end, wherein a first seal is fitted inside the second open end, and the capillary tube penetrates and is fixed to the first seal and extends into the storage tube through the second open end.

In other preferred solutions, the negative pressure tube is coaxially sleeved outside of the storage tube, and the starting device further comprises a second seal sandwiched between an outer wall of the storage tube and an inner wall of the negative pressure tube.

In other preferred solutions, a positioning part protruding from the outer wall of the storage tube is provided, an end of the negative pressure tube close to the connector abuts a side of the positioning part away from the first open end, and part of the second seal is sandwiched between the positioning part and the negative pressure tube.

In other preferred solutions, the connector comprises a pierceable seal that seals the first open end.

In other preferred solutions, the pierceable seal comprises a fixed part which is located outside the installation port end and has a size larger than a diameter of the storage tube; and a punctured part extending from the fixed part into the installation port end and matching the diameter of the storage tube.

In other preferred solutions, the connector further comprises a crown cover that is sleeved on an outer circumference of the fixed part and extends to an outer circumference of the storage tube.

In other preferred solutions, the fixed part is provided with an unobstructed recess extending to the punctured part, and when the connector is connected the mounting part, the first piercing piece pierces the punctured part through the unobstructed recess and is in communication with the storage tube.

This application provides an apparatus for collecting blood or other liquids in another aspect, characterized in that, the application comprises a housing, a starting device, a transmission device and a collection device. The housing is provided with an installation port and a collection port. The transmission device is housed in the housing, and at least part of the starting device is housed in the housing and connected to the transmission device through the installation port. At least part of the collection device is housed in the housing, and the collection device collects blood or other liquids through the collection port. The collection port and the installation port are not coaxially arranged.

In other preferred solutions, the housing comprises an upper housing and a lower housing, the lower housing is perforated with the collection port through which the collection device is extended, and the upper housing is perforated with the installation port for inserting the starting device.

In other preferred solutions, a projection of the collection port onto the upper housing along an extension direction of the collection device does not overlap with the installation port. In other preferred solutions, the transmission device comprises a locking assembly and a driving assembly, the driving assembly is connected to the collection device, at least part of the starting device is detachably connected to the locking assembly, and the locking assembly is configured to release a movement restriction on the driving assembly after the starting device is inserted, so that the driving assembly actuates the collection device to extend out of the housing, and the locking assembly is configured to restrict a movement of the driving assembly after the starting device is removed.

In other preferred solutions, the locking assembly comprises a pushing block and sliding pieces, the pushing block is movably connected to the sliding pieces, the pushing block is configured to move along a first direction under an action of the starting device and drive the sliding pieces, and the sliding pieces are configured to move along a second direction under an action of the pushing block, for restricting a movement of the collection device in the first direction and releasing a movement restriction on the collection device.

In other preferred solutions, the sliding piece comprises a first slider and a first deformable piece located between the first slider and an inner wall of the housing, the first deformable piece is connected to the first slider for reciprocating the first slider along the second direction.

In other preferred solutions, the sliding pieces are symmetrically arranged on both sides of the collection device along the second direction, and the pushing block is arranged between the two sliding pieces and pushes the two sliding pieces to move in opposite directions.

In other preferred solutions, the first slider has a body part and a position limiting part, the body part is connected to the first deformable piece, the position limiting part protrudes from the body part along the second direction, and the position limiting part is configured to at least partially stop the collection device in the first direction after the starting device is removed, and release a stop on the collection device after the starting device is inserted.

In other preferred solutions, the pushing block has an inserting-connection part and squeezing parts, the inserting-connection part is configured to form an inserting connection with the starting device, and the squeezing parts protrude from an outer side of the inserting-connection part for squeezing the sliding pieces in the second direction.

In other preferred solutions, a cross-sectional area of the squeezing part gradually decreases along an insertion direction of the starting device.

In other preferred solutions, the housing is provided with a guiding column;
the inserting-connection part comprises an inserting-connection section and a guiding section, the inserting-connection section is provided with an inserting-connection groove, the guiding section is sleeved on the guiding column, and the squeezing part is provided on the guiding section.

In other preferred solutions, the sliding piece is provided with a positioning portion corresponding to the squeezing parts, and the positioning portion is provided with accommodation parts that can accommodate the squeezing parts.

In other preferred solutions, the driving assembly is located between the collection device and an inner wall of the housing, the driving assembly comprises a driving shaft connected to the collection device and a precompressed driving elastic piece sleeved outside the driving shaft, and after the sliding pieces release the movement restriction on the collection device, the precompressed driving elastic piece pushes the driving shaft along the first direction and actuates the collection device to move towards outside of the housing to a collection position.

In other preferred solutions, the driving assembly further comprises a reset mechanism, which is configured to push the collection device to move towards inside of the housing after the collection device moves to the collection position, so as to reset the collection device.

In other preferred solutions, the reset mechanism comprises a shaft sleeve sleeved outside the driving shaft and a reset elastic piece connected to the shaft sleeve, and the driving shaft is configured to pass through the shaft sleeve along the second direction and has a protrusion protruding outward; after the collection device moves to the collection position, the reset elastic piece pushes the shaft sleeve and actuates the driving shaft and the collection device to move towards inside of the housing through the protrusion.

In other preferred solutions, an end of the reset elastic piece away from the collection device is fixed to the shaft sleeve, and an end of the reset elastic piece close to the collection device is configured to be movable relative to the shaft sleeve along the second direction relative to the housing.

In other preferred solutions, both the driving elastic piece and the reset elastic piece are precompressed springs, the reset elastic piece is sleeved on an outer circumference of the shaft sleeve, and an elastic coefficient of the driving elastic piece is greater than that of the reset elastic piece.

In other preferred solutions, the driving assembly further comprises a top position-limiting mechanism located at an end of the precompressed driving elastic piece away from the collection device, and the top position-limiting mechanism is configured to precompress the precompressed driving elastic piece and the reset elastic piece.

In other preferred solutions, the top position-limiting mechanism comprises second sliders located on opposite sides of the driving shaft and position-limiting elastic pieces for actuating two second sliders to move relative to each other, at least two second sliders enclose an unobstructed part, and an end of the driving shaft away from the collection device extends outside the top position-limiting mechanism through the unobstructed part and is spaced apart from the top position-limiting mechanism.

In other preferred solutions, the position-limiting elastic piece is a precompressed position-limiting elastic piece connected between the two second sliders which are arranged opposite to each other, and the two second sliders and the precompressed position-limiting elastic pieces enclose the unobstructed part; or
the position-limiting elastic piece is a pre-stretched position-limiting elastic piece connected between the slider and the housing, and the two second sliders are spaced to form the unobstructed part therebetween.

In other preferred solutions, a position-limiting groove is provided on a side of the second slider close to the collection device, and the position-limiting groove is configured to form a snapping connection with the shaft sleeve;
when the collection device moves to the collection position, the driving elastic piece drives the driving shaft to actuate the shaft sleeve to move and disengage from the position-limiting groove, the position-limiting elastic piece drives the two second sliders to separate, an end of the driving elastic piece away from the collection device is released through a gap generated by a separation between the second sliders, and the reset elastic piece pushes the shaft sleeve and actuates the driving shaft and the collection device to move towards inside of the housing through the protrusion.

In other preferred solutions, the first direction is perpendicular to the second direction.

In other preferred solutions, a first fluid channel is provided inside the housing, and the starting device is in communication with the collection device through the first fluid channel.

In other preferred solutions, the starting device comprises a starting assembly and a first puncturing assembly, the starting assembly is detachably connected to the locking assembly, the first puncturing assembly is detachably connected to the starting assembly, and the first puncturing assembly is configured to pierce the starting assembly when the starting assembly is inserted into the locking assembly of the transmission device, so that the starting assembly is in communication with the collection device via the first puncturing assembly.

In other preferred solutions, the starting assembly comprises a connector, a storage tube extending from the connector, and a negative pressure tube in communication with the storage tube, the connector is configured to be detachably connected to the locking assembly, the negative pressure tube has a pressure lower than atmospheric pressure, and the storage tube is configured to be in communication with the collection device through the first puncturing assembly when the connector is inserted into the locking assembly, for storing the blood or other liquids collected by the collection device.

In other preferred solutions, one of the first puncturing assembly and the starting assembly comprises a first piercing piece which has a hollow tubular shape with two open ends, and the other one comprises a first puncturable seal corresponding to the first piercing piece, and the first piercing piece is configured to pierce the first puncturable seal when the connector is inserted into the locking assembly, to establish communication between the storage tube and the collection device.

In other preferred solutions, the starting assembly further comprises a capillary tube connected to the storage tube, and the storage tube is in communication with the negative pressure tube through the capillary tube.

In other preferred solutions, the storage tube comprises a first open end connected to the connector, and a second open end opposite to the first open end, a first seal is fitted inside the second open end, and the capillary tube penetrates and is fixed to the first seal and extends into the storage tube through the second open end.

In other preferred solutions, the negative pressure tube is coaxially sleeved outside of the storage tube, and the starting assembly further comprises a second seal sandwiched between an outer wall of the storage tube and an inner wall of the negative pressure tube.

In other preferred solutions, a positioning part protruding from the outer wall of the storage tube is provided, an end of the negative pressure tube close to the connector abuts a side of the positioning part away from the first open end, and part of the second seal is sandwiched between the positioning part and the negative pressure tube.

In other preferred solutions, the starting assembly comprises a first puncturable seal that seals the first open end, the first puncturable seal comprises a fixed part which is located outside the open end and has a size larger than a diameter of the storage tube; and a punctured part extending from the fixed part into the open end and matching the diameter of the storage tube.

In other preferred solutions, the connector comprises a crown cover that is sleeved on an outer circumference of the fixed part and extends to an outer circumference of the storage tube.

In other preferred solutions, the fixed part is provided with an unobstructed recess corresponding to the first puncturable seal and extending to the punctured part, and when the connector is inserted into the locking assembly, the first piercing piece pierces the punctured part through the unobstructed recess and is in communication with the storage tube.

In other preferred embodiments, the collection device comprises a second puncturing assembly, a second puncturable seal, and a blood collection assembly, wherein at least part of the blood collection assembly is provided at the collection port and located within the housing, the second puncturable seal is provided between the housing and the blood collection assembly, and the second puncturing assembly is configured to extend out of the housing through the second puncturable seal under an action of the driving assembly.

In other preferred solutions, the blood collection assembly comprises a first base plate and a second base plate which are stacked along the first direction, wherein the first base plate and the second base plate are arranged outside the collection port and connected to the housing, the first base plate and the second base plate collectively form a collection chamber, and since the collection chamber is configured to establish communication between the storage tube and the collection device when the starting device is inserted into the locking assembly, the collection port and the storage tube are in communication internally and have a pressure lower than atmospheric pressure, to collect blood or other liquids.

In other preferred solutions, the second puncturable seal comprises a first sealing part, an elastic deformation part, and a second sealing part, the elastic deformation part is located between the first sealing part and the second sealing part, the first sealing part is configured to be pierced by the second puncturable seal, and the second sealing part is provided at the collection port.

In other preferred solutions, the elastic deformation part is formed as a hollow frustum-shaped structure in an initial state.

In other preferred solutions, the second puncturing assembly comprises a needle holder and at least one microneedle fixed to the needle holder, and the first base plate is provided with a through hole(s) corresponding to the at least one microneedle one by one.

In other preferred solutions, the first base plate is further provided with a second fluid channel, which is in communication with the collection chamber.

In other preferred solutions, at least part of the fluid channel is formed as a wavy structure.

In other preferred solutions, the second base plate is provided with a penetrated hole, the first base plate is provided with a central part protruding towards the penetrated hole, and the central part is provided in the penetrated hole and a gap is formed between the central part and the penetrated hole; the second puncturing assembly is provided at a position corresponding to the gap.

In other preferred solutions, a surface of the central part away from the first base plate is flush with a surface of the second base plate away from the first base plate.

This application provides a method for collecting blood or other liquids from a subject in yet another aspect, comprising:
applying a housing to a skin of the subject, wherein the housing is provided with a collection device therein, which is extendable and retractable relative to the housing, and a transmission device associated with the collection device;
inserting the starting device into the transmission device, wherein the transmission device drives the collection device to extend out of the housing along a first direction, to pierce the skin of the subject.

In other preferred solutions, the transmission device driving the collection device to extend out of the housing along the first direction, comprises:
the transmission device comprising a locking assembly and a driving assembly, wherein the driving assembly is connected to the collection device, and the starting device is detachably connected to the locking assembly;
the starting device driving the locking assembly to slide relative to the housing along a second direction, to release a movement restriction on the driving assembly;
the locking assembly actuating the driving assembly, which drives the collection device to move along the first direction towards outside of the housing.

In other preferred solutions, the locking assembly actuating the driving assembly, comprises:
the locking assembly moving from a first position to a second position along the second direction, an end of the locking assembly close to the driving assembly being provided with a position limiting part; in the first position, the position limiting part being located at a movement path of the collection device and limiting at least part of the collection device within the housing; in the second position, the position limiting part disengaging from the driving assembly and releasing the collection device.

In other preferred solutions, the starting device driving the locking assembly to slide relative to the housing along the second direction, comprises:
the locking assembly comprising a pushing block and sliding pieces, wherein the pushing block is configured to move along the first direction under an action of the starting device and drive the sliding pieces, and the sliding pieces are configured to move in the second direction under an action of the pushing block.

In other preferred solutions, the locking assembly actuating the driving assembly, which drives the collection device to move along the first direction towards outside of the housing, comprises:
after the driving assembly disengaging from the position limiting part, a driving elastic piece in the driving assembly pushing a driving shaft associated with the collection device, and actuating the collection device to move towards outside of the housing and pierce the skin of the subject.

In other preferred solutions, the method further comprises:
enabling the driving assembly to further drive the driving shaft and actuate a shaft sleeve sleeved outside the driving shaft and the collection device to move towards the skin to a blood collection position, thereby compressing a reset elastic piece sleeved outside the shaft sleeve;
enabling the compressed reset elastic piece to drive the shaft sleeve, which actuate the collection device to move from the blood collection position towards inside of the housing through the driving shaft, to move away from the skin of the subject.

In other preferred solutions, a collection port is provided on a side of the housing close to the collection device, and the transmission device drives the collection device to extend out of the housing through the collection port along the first direction, and the method further comprises:
after the starting device is inserted into the transmission device, enabling a negative pressure tube inside the starting device to be in communication with the collection port, the skin of the subject being sucked into the collection port by means of a pressure difference between the collection port and the negative pressure tube;
enabling the blood or other liquids which are generated after the skin is pierced by the collection device to flow into the negative pressure tube through the collection port.

This application provides a microneedle driving mechanism for driving a microneedle assembly that is extendable and retractable along a first direction in yet another aspect, comprising:
a housing;
a driving shaft, accommodated within the housing and connected to the microneedle assembly;
a driving elastic piece, sleeved on the driving shaft, wherein the driving elastic piece is located between an inner wall of the housing and the microneedle assembly;
a position limiting part, limited at a movement path of the microneedle assembly, wherein the position limiting part is driven by an external force to be movable relative to the housing;
wherein a movement direction of the position limiting part is not parallel to the first direction.

In other preferred solutions, the housing comprises a hollow lower housing supported at a side of the position limiting part away from the driving shaft, and a hollow upper housing fixed relative to the lower housing and limited at a side of the driving shaft away from the lower housing.

In other preferred solutions, the housing further comprises a circular support wall, which is sandwiched and fixed between the upper housing and the lower housing, and surrounds outer circumference of the driving shaft and the driving elastic piece.

In other preferred solutions, the position limiting part is sandwiched between the circular support wall and the lower housing along the first direction, and the lower housing is configured to be spaced apart from the position limiting part along a movement direction of the position limiting part.

In other preferred solutions, the microneedle driving mechanism further comprises a reset mechanism located between the driving elastic piece and the circular support wall, wherein the reset mechanism drives the microneedle assembly to move towards the upper housing, and the reset mechanism is separated from the driving elastic piece.

In other preferred solutions, the microneedle assembly comprises a needle holder and at least one microneedle connected to the needle holder.

In other preferred solutions, the needle holder is perforated with a counterbore along the first direction, and an end of the driving shaft is provided with a snapping part which axially extends and can be inserted and fixed in the counterbore.

In other preferred solutions, the snapping part comprises a snapping body connected to the driving shaft, and a shoulder connected to the snapping body, wherein the shoulder can approach or move away from a central axis of the driving shaft;
the counterbore penetrates the needle holder along the first direction, and the shoulder can pass through the counterbore and abut a side of the needle holder away from the driving shaft.

In other preferred embodiments, the side of the needle holder away from the driving shaft is provided with a snapping groove that can accommodate the snapping shoulder, and the snapping groove is recessed into the needle holder. When the snapping body passes through the counterbore, the shoulder can be snapped into the snapping groove.

Compared with the prior art, this application has at least the following beneficial effects:
The apparatus for collecting blood or other liquids provided in this application includes a housing, a starting device, a transmission device and a collection device, wherein the transmission device includes a locking assembly and a driving assembly that cooperate with each other; the locking assembly can lock and unlock the driving assembly; the driving assembly is connected to the collection device and is configured to drive the collection device to extend and retract; when the locking assembly is in an unlocked state, the restriction on the driving assembly is released, and the collection device extends; the transmission device and the collection device are both provided inside the housing; the starting device is independent of the housing and is detachably connected to the locking assembly; after the starting device is inserted, the locking assembly releases the movement restriction on the driving assembly, so that the driving assembly actuates the collection device to extend out of the housing; after the starting device is removed, the locking assembly restricts the movement of the driving assembly, to prevent the collection device from extending, ensuring that the collection device will not extend and injure a human body due to accidental contact, thereby improving the safety performance of the apparatus.

The starting device in this application is detachably connected to the locking assembly, and the collection device is extendable only when the starting device is inserted into the locking assembly, avoiding the collection device from extending due to accidental contact or activation, thereby effectively solving the problem of blood collection apparatuses in the prior art that are prone to injury to a human body caused by extension of blood collection components due to accidental activation or contact.

### BRIEF DESCRIPTION OF DRAWINGS

In order to provide a clearer explanation of technical solutions in the specific embodiments of this application or in the existing technology, a brief introduction to the drawings required for describing the specific embodiments or existing technology will be given below. It is obvious that the drawings in the following description relate to some embodiments of this application, and other drawings can be obtained based on these drawings for those skilled in the art without any creative labor.
Figure 1 is a perspective view of an apparatus for collecting blood or other liquids provided in this application;
Figure 2 is a top view of the apparatus for collecting blood or other liquids in Figure 1;
Figure 3 is a schematic view of an internal structure of the apparatus for collecting blood or other liquids provided in this application;
Figure 4 is a specific structural schematic view of a transmission device provided in this application;
Figure 5 is another specific structural schematic view of the transmission device provided in this application;
Figure 6 is a specific structural schematic view of a sliding piece provided in this application;
Figure 7 is a schematic view of an internal structure of a starting device provided in this application;
Figure 8 is a schematic view of an internal structure of a starting device provided in this application;
Figure 9 is another schematic view of an internal structure of the starting device provided in this application;
Figure 10A is a schematic sectional view of the apparatus for collecting blood or other liquids shown in Figure 2 taken along A-A line;
Figure 10B is a partial schematic view of the apparatus for collecting blood or other liquids shown in Figure 10A;
Figure 11 is a schematic sectional view of the apparatus for collecting blood or other liquids shown in Figure 2 taken along A-A line;
Figure 12 is a schematic perspective view of a sliding piece provided in this application;
Figure 13 is a schematic perspective view of a driving assembly provided in this application;
Figure 14 is another schematic perspective view of the driving assembly provided in this application;
Figure 15 is another schematic exploded structural view of a collection device provided in this application;
Figure 16 is a structural schematic view of a driving shaft in B-B section of Figure 2;
Figure 17 is a structural schematic view of a housing provided in this application;
Figure 18 is a schematic view of an internal structure of a starting device provided in this application;
Figure 19 is a specific structural schematic view of a connector provided in this application;
Figure 20 is a structural schematic view of a blood collection device in its non-triggered state provided in this application;
Figure 21 is a structural schematic view of the blood collection device in its triggered state provided in this application.

In the above drawings, the list of components represented by each reference number is as follows:
100, housing; 100A, upper housing; 100B, circular support wall; 100C, lower housing; 100a, bracket; 101, guiding column; 102, first puncturing assembly; 103, first piercing piece; 104, first puncturable seal 104;
200, starting device; 210, negative pressure tube; 220, connector; 220A, fixed part; 220B, punctured part; 220C, unobstructed recess; 221, crown cover; 230, storage tube; 231, first open end; 232, second open end; 233, first seal; 234, second seal; 235, positioning part; 240, capillary tube;
300, transmission device;
310, locking assembly; 311, pushing block; 3111, inserting-connection part; 3111A, inserting-connection section; 3111B, guiding section; 3111C, inserting-connection groove; 3112, squeezing part; 312, sliding piece; 3121, first slider; 3121A, body part; 3121B, position limiting part; 3121C, positioning portion; 3121D, accommodation part; 3122, first deformable piece;
320, driving assembly; 321, driving shaft; 321A, protrusion; 321B, snapping body; 321C, shoulder; 322, driving elastic piece; 323, reset mechanism; 3231, shaft sleeve; 3232, reset elastic piece; 3233, abutting part; 3234, mounting piece; 324, top position-limiting mechanism; 3241, unobstructed part; 3242, second slider; 32421, position-limiting groove; 3243, precompressed position-limiting elastic piece;
400, collection device; 401, collection port; 402, installation port; 4021, first fluid channel; 4022, second fluid channel; 411, second puncturing assembly; 411A, microneedle; 411B, needle holder; 411C, counterbore; 411D, snapping groove;
412, blood collection assembly; 412A, second base plate; 412B, first base plate; 4121, contact face; 4122, recess; 4122A, bottom wall; 4122B, surrounding wall; 4122C, central part; 4122D, through hole; 413, second puncturable seal; 413A, first sealing part; 413B, elastic deformation part; 413C, second sealing part;
500, barrier.

### DESCRIPTION OF EMBODIMENTS

In order to make the above and other features and advantages of this application clearer, this application is further described below in conjunction with the drawings. It should be understood that the specific embodiments given herein are for the purpose of explanation to those skilled in the art, only illustrative but not restrictive.

In the description of this application, it is to be understood that orientation or position relationships indicated by terms "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential" or the like are orientation or position relationships shown on the basis of the drawings, and the orientation or position relationships are not intended to indicate or imply that the referred device or element must be at the specific orientation or be constructed and operated at the specific orientation, but only intended for the convenience of describing this application and simplifying the description, and thus such orientation or position relationships may not be understood as limits to this application.

In addition, terms "first" and "second" are only adopted for the objective of description, and cannot be understood to indicate or imply relative importance of the indicated technical features or implicitly indicate the number of the indicated technical features. Therefore, a feature limited by "first" or "second" may explicitly or implicitly comprise at least one such feature. In the description of this application, unless otherwise explicitly and specifically limited, "multiple" means at least two, such as two, three, etc.

In this application, unless otherwise explicitly specified and limited, terms such as "mount", "connect", "connection" and "fix" should be broadly understood, for example, they may refer to a fixed connection, and may also refer to a detachable connection or being integrated; they may refer to a mechanical connection or may also refer to an electrical connection; they may refer to a direct connection, or may also refer to an indirect connection through an intermediate; they may refer to communication inside two elements or interaction relationship of two elements, unless otherwise explicitly limited. For ordinary persons skilled in the art, the specific meanings of the above terms in this application may be understood according to actual conditions.

In this application, unless otherwise explicitly specified and limited, the expression of a first feature being "over" or "under" a second feature may mean a direct contact between the first and second features, or an indirect contact between the first and second features through an intermediate. Moreover, the expression of a first feature being "over", "above" or "on top of" a second feature may mean that the first feature is directly above or obliquely above the second feature, or only represents that the horizontal height of the first feature is higher than that of the second feature. The expression of a first feature being "under", "below" or "underneath" a second feature may mean that the first feature is directly below or obliquely below the second feature, or only represents that the horizontal height of the first feature is lower than that of the second feature.

In the description of this specification, the description with reference to the terms "one embodiment", "some embodiments", "example", "specific example", or "some examples" or the like means that a specific feature, structure, material, or characteristic described in conjunction with the embodiment or example is comprised in at least one embodiment or example of this application. In this specification, exemplary description of the above terms is not necessarily referring to the same embodiment or example. Furthermore, the described specific features, structures, materials, or characteristics may be combined in a suitable manner in any one or more embodiments or examples. In addition, those skilled in the art may combine and incorporate different embodiments/examples or features of different embodiments/examples described in this specification unless they are inconsistent with each other.

### Embodiment 1

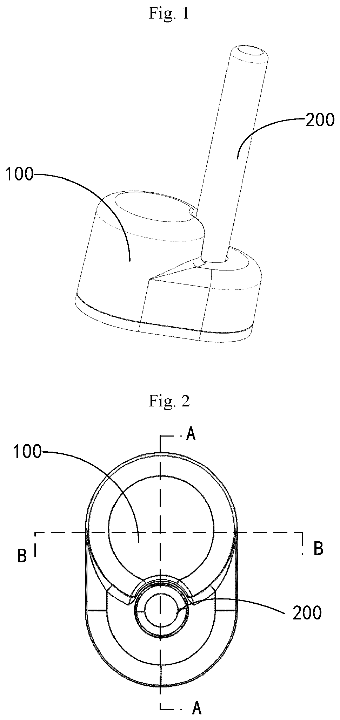

As shown in Figures 1-3, this application provides an apparatus for collecting blood or other liquids in a first aspect, comprising a housing 100, a starting device 200, a transmission device 300, and a collection device 400.

The collection device 400 is housed in the housing 100 and is configured to pierce a barrier object (such as skin) and collect blood or other liquids.

The transmission device 300 is housed in the housing 100 and includes a locking assembly 310 and a driving assembly 320. The driving assembly 320 is connected to the collection device 400 and is configured to control extending and retracting of the collection device 400 to complete collection action. The starting device 200 is detachably connected to the locking assembly 310, and the locking assembly 310 is configured to restrict the movement of the driving assembly 320 before the starting device 200 is inserted, and to release the movement restriction on the driving assembly 320 after the starting device 200 is inserted, so that the driving assembly 320 actuates the collection device 400 to extend out of the housing 100. That is, when the starting device 200 is not inserted into the locking assembly 310, the apparatus is in an unused state, and at this time, the locking assembly 310 will lock the driving assembly 320, preventing the driving assembly 320 from driving the collection device 400 to extend, thereby ensuring the safety of the apparatus. When the starting device 200 is inserted into the locking assembly 310, the locking assembly 310 releases the restriction on the driving assembly 320, allowing the driving assembly 320 to drive the collection device 400 to extend out of the housing 100, thereby completing collection of blood or other liquids.

The apparatus for collecting blood or other liquids in this solution includes a housing 100, a starting device 200, a transmission device 300, and a collection device 400. The transmission device 300 includes a locking assembly 310 and a driving assembly 320 which cooperate with each other. The locking assembly 310 can lock and unlock the driving assembly 320. The driving assembly 320 is connected to the collection device 400 and is configured to drive the collection device 400 to extend and retract. When the locking assembly 310 is in an unlocked state, the restriction on the driving assembly 320 is released, and the collection device 400 extends. The transmission device 300 and the collection device 400 are both provided within the housing 100. The starting device 200 is independent of the housing 100 and can be detachably connected to the locking assembly 310. After the starting device 200 is inserted, the locking assembly 310 releases the movement restriction on the driving assembly 320, so that the driving assembly 320 actuates the collection device 400 to extend out of the housing 100, and after the starting device 200 is removed, the locking assembly 310 restricts the movement of the driving assembly 320 to prevent the collection device 400 from extending, ensuring that the collection device 400 will not extend and injure a human body due to accidental contact, thereby improving the safety performance of the apparatus.

In this solution, the starting device 200 is detachably connected to the locking assembly 310. The collection device 400 can only extend when the starting device 200 is inserted into the locking assembly 310, which will not cause the collection device 400 to extend due to accidental contact or activation. This effectively solves the problem of blood collection apparatuses in the prior art that are prone to injury to a human body caused by extension of blood collection components due to accidental activation or contact.

### Embodiment 2

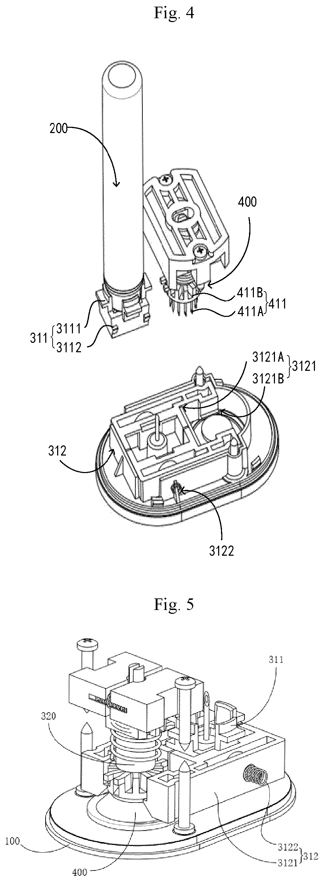

Furthermore, as shown in Figures 4 and 5, Embodiment 2 is a further improvement based on Embodiment 1. The locking assembly 310 includes a pushing block 311 and sliding pieces 312. The pushing block 311 is movable relative to the sliding pieces 312, and the movement of the pushing block 311 by itself can actuate the sliding pieces 312 to move. This embodiment does not limit how the pushing block 311 drives the sliding pieces 312 to move. The pushing block 311 can be configured to directly contact and abut the sliding pieces 312, or indirectly drive the sliding pieces 312 to move through an intermediate.

The pushing block 311 is configured to move along a first direction under the action of the starting device 200 and drive the sliding pieces 312. The sliding pieces 312 are configured to move along a second direction under the action of the pushing block 311, for restricting the movement of the collection device 400 in the first direction and release the movement restriction on the collection device 400. The sliding pieces 312 can restrict the movement of the collection device 400 in the first direction, which is the direction in which the collection device 400 pierces a barrier. When the sliding pieces 312 are in a certain locking position, they can restrict the movement of the collection device 400 in the first direction, that is, limit the extension of the collection device 400. When the sliding pieces 312 move along the second direction under the action of the pushing block 311 to another unlocking position, they can release the movement restriction on the collection device 400 in the first direction, so that the collection device 400 can move along the first direction and extend out of the housing 100, and then complete the collection action.

In this solution, the sliding pieces 312 and the collection device 400 can be limited in position by a buckle-like structure, and when the sliding pieces 312 move along the second direction under the action of the pushing block 311 to another unlocking position, they separates at the buckle, releasing the position limitation on the driving assembly 320 and allowing it to drive the collection device 400 to extend.

When the starting device 200 is inserted, the pushing block 311 is subjected to force and moves along the first direction. At this time, the sliding pieces 312 will move along the second direction under the action of the pushing block 311 to move away from the collection device 400, thereby releasing the movement restriction of the sliding pieces 312 on the collection device 400 in the first direction. In this solution, the first direction and the second direction are not collinear, which can make the layout of the entire apparatus more reasonable and avoid excessive elongation of the apparatus.

Optionally, as shown in Figure 5, the sliding piece 312 includes a first slider 3121 and a first deformable piece 3122 located between the first slider 3121 and an inner wall of the housing 100 (only the bottom of the housing 100 is shown in Figure 5). The first deformable piece 3122 is connected to the first slider 3121 and is configured for reciprocating the first slider 3121 along the second direction. When the starting device 200 is inserted, it will actuate the first slider 3121 to squeeze and compress the first deformable piece 3122. When the starting device 200 is removed, the first deformable piece 3122 will return to its original state and actuate the first slider 3121 to reset, thereby re-limiting the position of the driving assembly 320.

Optionally, as shown in Figure 5, in this embodiment, sliding pieces 312 are symmetrically arranged on both sides of the collection device 400 to make the structure more stable and the internal layout of the apparatus more symmetrical and reasonable. The pushing block 311 is provided between the two sliding pieces 312 and pushes them to move in opposite directions. The sliding pieces 312 are symmetrically arranged relative to the pushing block 311, this layout is more reasonable, and the pushing block 311 actuates the sliding pieces 312 to move synchronously toward opposite sides, which is more stable than single-side movement. The first deformable piece 3122 can be a spring. When the starting device 200 is inserted, the pushing block 311 will squeeze the pair of first sliders 3121 on its outside in the second direction. Due to the compression of the spring being subject to pressure, the first sliders 3121 will move away from each other, thereby releasing the restriction on the driving assembly 320, and the driving assembly 320 will thereby drive the collection device 400 to extend out of the housing 100 along the first direction.

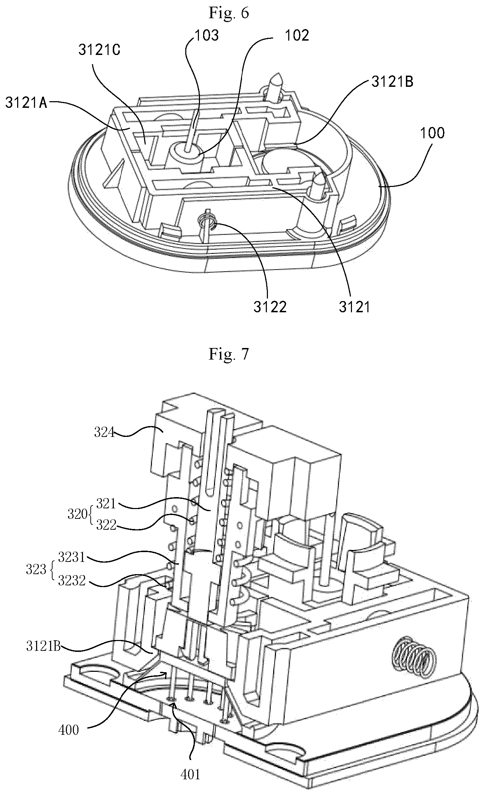

Furthermore, a more specific definition is made on the structure of the first slider 3121, as shown in Figures 4 and 6. The first slider 3121 has a body part 3121A and a position limiting part 3121B. The body part 3121A is connected to the first deformable piece 3122, and the position limiting part 3121B protrudes from the body part 3121A along the second direction. The position limiting part 3121B is configured to at least partially stop the collection device 400 in the first direction after the starting device 200 is removed, and release the stop on the collection device 400 after the starting device 200 is inserted. The position limiting effect on the collection device 400 is achieved through the position limiting part 3121B. Specifically, when the starting device 200 is not inserted, the collection device 400 is in a retracted state, and when the starting device 200 is inserted, the position limiting part 3121B releases the position limiting effect on the collection device 400, thereby allowing the collection device 400 to extend.

Optionally, the position limiting part 3121B is an inverted triangular block. Before the starting device 200 is inserted, an upper part of the inverted triangular block abuts the driving assembly 320, thereby preventing the driving assembly 320 from driving the collection device 400 to extend; after the starting device 200 is inserted, the inverted triangular block will separate from the collection device 400, thereby releasing the restriction on the driving assembly 320, and thus allowing the driving assembly 320 to drive the collection device 400 to extend along the first direction and pierce the barrier.

### Embodiment 3

As shown in Figure 4, this embodiment provides a more specific definition on the structure of the pushing block 311. The pushing block 311 has an inserting-connection part 3111 and squeezing parts 3112. The inserting-connection part 3111 is configured to form an inserting connection with the starting device 200, and the inserting-connection part 3111 can be a groove-shaped structure that can accommodate the starting device 200. The squeezing parts 3112 are provided in pairs and protrude from an outer side of the inserting-connection part 3111, for squeezing the sliding piece 312 in the second direction. When the starting device 200 is inserted into the inserting-connection part 3111 of the pushing block 311, the two squeezing parts 3112 of the pushing block 311 will respectively squeeze the two sliding pieces 312 in the second direction, and the position limiting part 3121B of the sliding pieces 312 will disengage from the collection device 400, so as to release the position limiting effect on the driving assembly 320.

Optionally, the cross-sectional area of the squeezing part 3112 gradually decreases along the insertion direction of the starting device 200, thereby facilitating the insertion of the starting device 200.

In this embodiment, the squeezing part 3112 is narrow at a lower end and wide at an upper end, wherein the narrower lower end facilitates insertion of the starting device 200 into the inserting-connection part 3111, and the wider upper end is configured for squeezing the sliding piece 312. Optionally, the squeezing part 3112 is an inverted right angled triangle structure, and its oblique edge squeezes the inserting-connection part 3111 and moves the inserting-connection part 3111, thereby pushing the sliding piece 312.

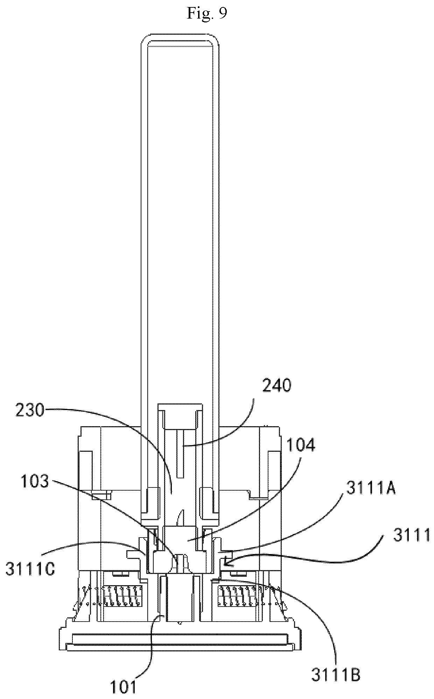

This embodiment further supplements the structure of the housing 100, which is provided with a guiding column 101. As shown in Figure 9, the inserting-connection part 3111 includes an inserting-connection section 3111A and a guiding section 3111B. The inserting-connection section 3111A is provided with an inserting-connection groove 3111C, and the guiding section 3111B is sleeved on the guiding column 101 to facilitate the installation of the guiding section 3111B and avoid errors that may affect accuracy. The squeezing part 3112 is provided on the guiding section 3111B, and specifically, it can be understood as the squeezing part 3112 being connected to the guiding section 3111B and integrally formed on an outside of the guiding section 3111B for squeezing the sliding piece 312. When the starting device 200 is inserted, the squeezing part 3112 outside the guiding section 3111B squeezes the sliding piece 312 in the second direction, causing the position limiting part 3121B to release its position limiting effect on the driving assembly 320, thereby allowing the collection device 400 to extend.

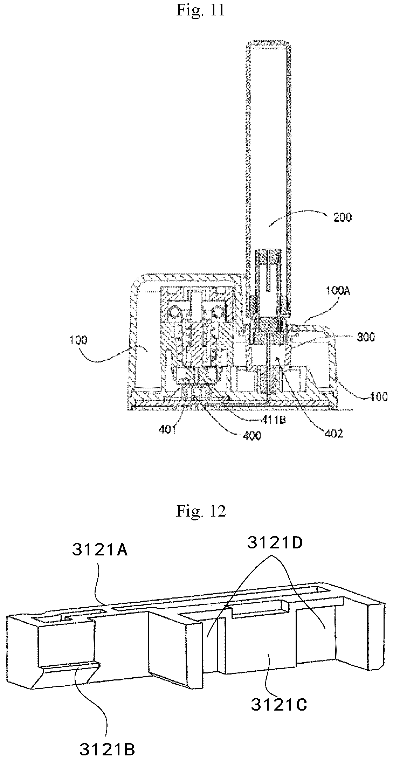

Furthermore, as shown in Figure 12, a further improvement has been made to the sliding piece 312. The sliding piece 312 is provided with a positioning portion 3121C corresponding to the squeezing parts 3112. The positioning portion 3121C is provided with accommodation parts 3121D that can accommodate the squeezing parts 3112, which are configured to limit the movement of the squeezing parts 3112. When the starting device 200 is inserted, the squeezing part 3112 squeezes the sliding piece 312. When the squeezing part 3112 moves to the lowest point, the squeezing part 3112 can enter the accommodation part 3121D and abut the positioning portion 3121C, which indicates that the starting device 200 is inserted in place.

### Embodiment 4

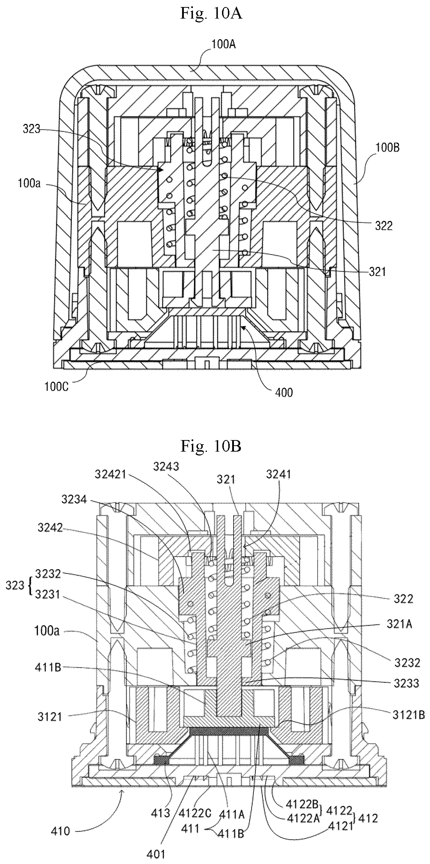

As shown in Figures 7 and 10A-10B, this embodiment is a further improvement of Embodiment 2. The driving assembly 320 is located between the collection device 400 and the inner wall of the housing 100, and is configured to drive the collection device 400 to extend from or retract into the housing 100. There is also a bracket 100a inside the housing 100 for supporting the driving assembly 320, as shown in Figure 10A, and the internal bracket 100a is fixed to a lower housing 100c through a structure such as a screw, the internal bracket 100a is provided with a space for installing the driving assembly 320 therein. Specifically, when the starting device 200 is inserted into the housing 100, the sliding pieces 312 are squeezed to move away from each other, and at this time, the position limiting part 3121B on the sliding pieces 312 can release the movement restriction on the driving assembly 320, and thus the driving assembly 320 can drive the collection device 400 to extend. The driving assembly 320 includes a driving shaft 321 connected to the collection device 400 and a driving elastic piece 322 sleeved outside the driving shaft 321. When the sliding pieces 312 move to a state where the position limiting part 3121B disengages from the driving assembly 320, the sliding pieces 312 release the movement restriction on the collection device 400, and the precompressed driving elastic piece 322 pushes the driving shaft 321 along the first direction and actuates the collection device 400 to move towards outside of the housing 100 to a collection position. In this embodiment, the first direction in which the collection device 400 extends is parallel to the direction in which the starting device 200 is inserted.

### Embodiment 5

As shown in Figures 7 and 10A-10B, in this embodiment, the driving assembly 320 includes not only the driving shaft 321 connected to the collection device 400 and the driving elastic piece 322 sleeved outside the driving shaft 321, but also a reset mechanism 323. After the collection device 400 moves to the collection position, the reset mechanism 323 pushes the collection device 400 to move towards inside of the housing 100. The reset mechanism 323 is separated from the driving elastic piece 322, that is, the driving assembly 320 can drive the collection device 400 to extend for collecting blood or other liquids, and can also drive the collection device 400 to retract after the collection is completed, so as to ensure sampling safety.

Optionally, the reset mechanism 323 includes a shaft sleeve 3231 sleeved outside the driving shaft 321 and a reset elastic piece 3232 connected to the shaft sleeve 3231. The driving shaft 321 is configured to pass through the shaft sleeve 3231 along the first direction and has a protrusion 321A protruding outward, that is, the driving shaft 321 is provided inside the shaft sleeve 3231, and the shaft sleeve 3231 is provided inside the reset elastic piece 3232. After the collection device 400 moves to the collection position, the reset elastic piece 3232 pushes the shaft sleeve 3231 and actuates the driving shaft 321 and the collection device 400 to move towards inside of the housing 100 through the protrusion 321A.

Optionally, in other preferred embodiments, a bottom of the shaft sleeve 3231 is provided with an abutting part 3233 which extends inward and can abut the protrusion 321A. When the reset elastic piece 3232 tends to reset and rebounds, the abutting part 3233 will abut the protrusion 321A and actuate the protrusion 321A to move in the direction in which the reset elastic piece 3232 rebounds, that is, actuate the driving shaft 321 to move in the direction in which the reset elastic piece 3232 rebounds, thereby moving the collection device 400 towards inside of the housing 100.

Optionally, the end of the reset elastic piece 3232 away from the collection device 400 is fixed to the shaft sleeve 3231, and the end of the reset elastic piece 3232 close to the collection device 400 is configured to be movable relative to the shaft sleeve 3231 along the first direction.

As shown in Figure 10B, an upper half of the shaft sleeve 3231 is provided with a mounting piece 3234 protruding outward. One end of the reset elastic piece 3232 is fixed inside the mounting piece 3234, and the other end is configured to be movable relative to the shaft sleeve 3231 along the first direction. When the other end moves in the direction opposite to the first direction, it can actuate the shaft sleeve 3231 to move in the same direction, and then actuate the driving shaft 321 to move towards inside of the housing 100 through the protrusion 321A, so that the collection device 400 is retracted into the housing 100.

### Embodiment 6

As shown in Figure 10B, this embodiment proposes a better choice for the driving elastic piece 322 and the reset elastic piece 3232. Both the driving elastic piece 322 and the reset elastic piece 3232 are precompressed springs, which can increase the load capacity of the springs. The reset elastic piece 3232 is sleeved on an outer circumference of the shaft sleeve 3231. The elastic coefficient of the driving elastic piece 322 is greater than that of the reset elastic piece 3232, so that, after the position limiting part 3121B releases the restriction on the collection device 400, the elasticity of the driving elastic piece 322 can resist the elasticity of the reset elastic piece 3232, and then the collection device 400 extends.

### Embodiment 7

As shown in Figure 7, in order to better understand how the driving elastic piece 322 works, this embodiment provides a feasible solution. In this embodiment, the driving assembly 320 also includes a top position-limiting mechanism 324 located at the end of the driving elastic piece 322 away from the collection device 400. The top position-limiting mechanism 324 is configured to precompress the driving elastic piece 322, that is, the driving elastic piece 322 is located between the protrusion 321A on the side wall of the driving shaft 321 and the top position-limiting mechanism 324. When the position limiting part 3121B releases the position limiting effect on the collection device 400, the driving shaft 321 will move along the first direction under the action of the driving elastic piece 322 and drive the collection device 400 to extend out of the housing 100. When the top position-limiting mechanism 324 releases the precompressing effect on the driving elastic piece 322, the driving elastic piece 322 will move towards the top position-limiting mechanism 324.

### Embodiment 8

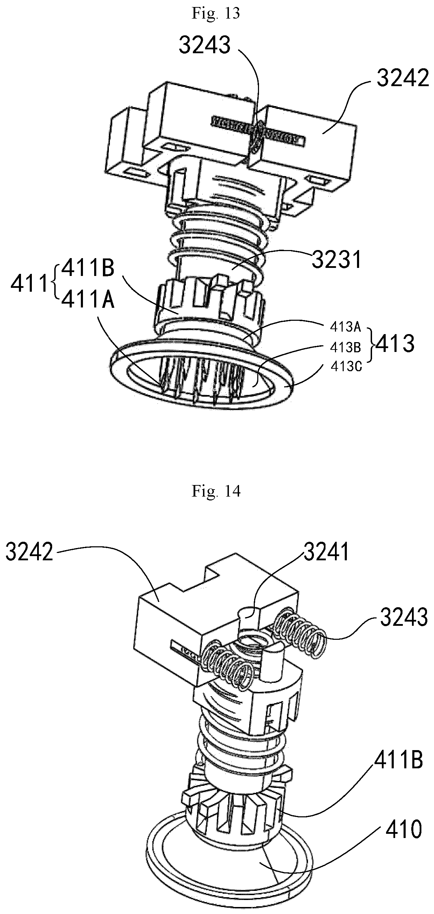

As shown in Figures 10B and 14, this embodiment provides a specific implementation for the cooperation between the top position-limiting mechanism 324 and the driving shaft 321, which can be combined with other embodiments. In this embodiment, the top position-limiting mechanism 324 is provided with an unobstructed part 3241 corresponding to the driving shaft 321. The end of the driving shaft 321 away from the collection device 400 can pass through the unobstructed part 3241, and the end of the driving shaft 321 away from the collection device 400 extends outside the top position-limiting mechanism 324 through the unobstructed part 3241 and is spaced apart from the top position-limiting mechanism 324.

In some embodiments, a more detailed description of the specific layout of the top position-limiting mechanism 324 is provided. The top position-limiting mechanism 324 includes second sliders 3242 located on opposite sides of the driving shaft 321 and spaced apart from each other, as well as precompressed position-limiting elastic pieces 3243 connected between the two second sliders 3242 that are arranged to be opposite. The top sliders and the precompressed position-limiting elastic pieces 3243 enclose the unobstructed part 3241. A position-limiting groove 32421 is provided on the side of the second slider 3242 close to the collection device 400, which is configured to form a snapping connection with the shaft sleeve 3231. Specifically, the side of the shaft sleeve 3231 away from the collection device 400 forms the snapping connection with the position-limiting groove 32421 to limit the movement of the two second sliders 3242. When the shaft sleeve 3231 moves along the first direction under the action of the driving elastic piece 322, the shaft sleeve 3231 will disengage from the position-limiting groove 32421, thereby releasing the position limiting effect on the two second sliders 3242.

When the collection device 400 moves to the collection position, the driving elastic piece 322 drives the driving shaft 321 to actuate the shaft sleeve 3231 to move and disengage from the position-limiting groove 32421. The precompressed position-limiting elastic piece 3243 drives the two second sliders 3242 to separate, and then the gap of the unobstructed part 3241 increases. The end of the driving elastic piece 322 away from the collection device 400 is released through the gap generated by the separation between the second sliders 3242, and at this time, the reset elastic piece 3232 pushes the shaft sleeve 3231 and actuates the driving shaft 321 and the collection device 400 to move towards inside of the housing 100 through the protrusion 321A, thereby resetting the collection device 400.

### Embodiment 9

In this embodiment, a structure different from the top position-limiting mechanism 324 in Embodiment 8 is provided. The top position-limiting mechanism 324 includes second sliders 3242 located on opposite sides of the driving shaft 321 and a pre-stretched position-limiting elastic piece (not shown in the drawings) connected between the slider and the housing 100, wherein an unobstructed part 3241 is formed between the second sliders 3242.

In the initial state, the pre-stretched position-limiting elastic piece is in an extended and stretched state. A position-limiting groove 32421 is provided on the side of the second slider 3242 close to the collection device 400, which is configured to form a snapping connection with the shaft sleeve 3231. Specifically, the side of the shaft sleeve 3231 away from the collection device 400 forms the snapping connection with the position-limiting groove 32421 to limit the movement of the two second sliders 3242. When the shaft sleeve 3231 moves along the first direction under the action of the driving elastic piece 322, the shaft sleeve 3231 will disengage from the position-limiting groove 32421, thereby releasing the position limiting effect on the two second sliders 3242.

When the collection device 400 moves to the collection position, the driving elastic piece 322 drives the driving shaft 321 to actuate the shaft sleeve 3231 to move and disengage from the position-limiting groove 32421. The pre-stretched position-limiting elastic piece drives the two second sliders 3242 to separate, and then the gap of the unobstructed part 3241 increases. The end of the driving elastic piece 322 away from the collection device 400 is released through the gap generated by the separation between the second sliders 3242, and at this time, the reset elastic piece 3232 pushes the shaft sleeve 3231 and actuates the driving shaft 321 and the collection device 400 to move towards inside of the housing 100 through the protrusion 321A, thereby resetting the collection device 400.

### Embodiment 10

To ensure a more compact overall structure of the apparatus, in this embodiment, the first direction is configured to be perpendicular to the second direction, and components, such as the pushing block 311 and the sliding pieces 312, are symmetrically distributed inside the housing 100. The layout is more reasonable.

Optionally, the first direction is parallel to the direction in which the collection device 400 pierces the barrier, and the second direction is parallel to the tangent plane of the barrier surface, to ensure piercing the barrier orthogonally and thus ensure the collection effect.

### Embodiment 11

As shown in Figures 7 and 11, this embodiment further improves how the collection device 400 achieves the collection function. Specifically, a collection port 401 is provided at the side of the housing 100 close to the collection device 400, and the transmission device 300 drives the collection device 400 along the first direction to extend out of the housing 100 through the collection port 401.

After the starting device 200 is inserted, the locking assembly 310 releases the movement restriction on the driving assembly 320, so that the driving assembly 320 actuates the collection device 400 to extend out of the housing 100 through the collection port 401. In the state where the starting device 200 is not inserted, the locking assembly 310 restricts the movement of the driving assembly 320, which makes it impossible for the collection device 400 to extend, ensuring that the collection device 400 will not extend and injure a human body due to accidental contact, thereby improving the safety performance of the apparatus.

When the starting device 200 is inserted into the housing 100, the sliding pieces 312 are squeezed to move away from each other, and at this time, the position limiting part 3121B releases the movement restriction on the driving assembly 320, and then the driving assembly 320 can drive the collection device 400 to extend from the collection port 401. The driving assembly 320 includes a driving shaft 321 connected to the collection device 400 and a driving elastic piece 322 sleeved outside the driving shaft 321. When the sliding pieces 312 move to a state where the position limiting part 3121B disengages from the driving assembly 320, the sliding pieces 312 release the movement restriction on the collection device 400, and the driving elastic piece 322 pushes the driving shaft 321 along the first direction and actuates the collection device 400 to move towards outside of the housing 100 to the collection position through the collection port 401. After piercing the barrier, the collection device enters the housing 100 through the collection port 401 again. In this embodiment, the first direction in which the collection device 400 extends is parallel to the direction in which the starting device 200 is inserted.

### Embodiment 12

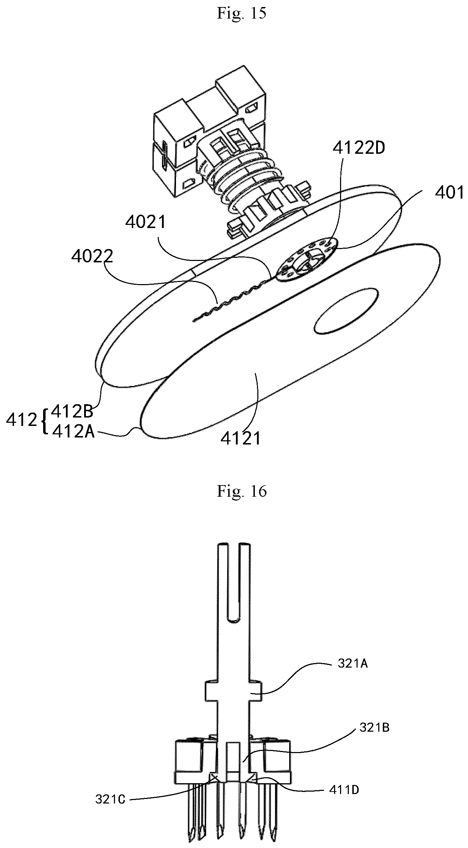

As shown in Figure 15, in order to ensure the collection effect and timely recover and store the collected blood or other liquids, it is necessary to transfer the liquid collected by the collection device 400 in a timely manner. To achieve this goal, this application provides a first fluid channel 4021 and a second fluid channel 4022 in communication with the collection port 401 in the housing 100. The second fluid channel 4022 can be provided in a snake curved shape in the housing 100. The starting device 200 is in communication with the collection port 401 through the second fluid channel 4022. After the starting device 200 is inserted into the locking assembly 310, the locking assembly 310 releases the movement restriction on the driving assembly 320, so that the driving assembly 320 actuates the collection device 400 to extend out of the housing 100 through the collection port 401 and pierce the barrier. After the liquid inside the barrier enters the collection device 400 through the collection port 401, it will promptly flow into the first fluid channel 4021 and the second fluid channel 4022 in sequence, and then flow into the starting device 200. The starting device 200 can store the collected liquid in a timely manner. When the starting device 200 is removed, the transfer of the liquid stored inside the starting device 200 can be achieved.

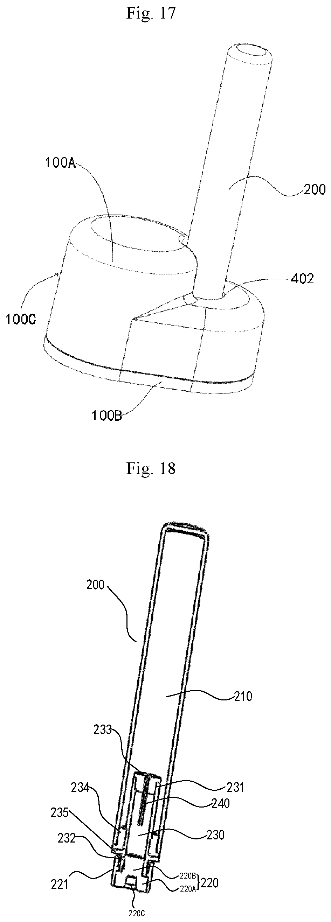

Optionally, as shown in Figure 18, in order to ensure smooth collection of liquid from the barrier, this embodiment provides a specific structure of the starting device 200. The starting device 200 includes a negative pressure tube 210, which has a pressure lower than atmospheric pressure. When the starting device 200 is inserted into the transmission device 300, the negative pressure tube 210 is in communication with the collection port 401 through the second fluid channel 4022. Through the negative pressure suction inside the negative pressure tube 210, the liquid inside the barrier can be sucked reversely into the collection device 400 via the collection port 401, and then flow into the starting device 200 via the second fluid channel 4022 to achieve liquid collection.

### Embodiment 13

As shown in Figures 10A-10B, this embodiment provides a specific structure of the collection device 400. The collection device 400 includes a second puncturing assembly 411 which is at least partially accommodated within the housing 100. The position of the lower housing 100C corresponding to a contact barrier 500 is provided with a blood collection assembly 412, and the collection port 401 is provided at the blood collection assembly 412; the second puncturing assembly 411 is movable relative to the blood collection assembly 412 and is configured to pierce the barrier through the collection port 401. That is, when the starting device 200 is inserted into the housing 100, the driving assembly 320 can drive the second puncturing assembly 411 to penetrate through the collection port 401 and pierce the barrier 500.

The blood collection assembly 412 includes a contact face 4121 close to the barrier 500. When collecting is performed, the contact face 4121 is in close contact with the barrier, and the contact face 4121 is recessed in the direction away from the barrier to form a recess 4122. The recess 4122 includes a bottom wall 4122A opposite to the barrier and a surrounding wall 4122B which extends from the bottom wall 4122A towards the barrier and is connected between the contact face 4121 and the bottom wall 4122A. The bottom wall 4122A is provided with a central part 4122C which protrudes towards the barrier and is spaced apart from the surrounding wall 4122B, the collection port 401 is provided around an outer circumference of the central part 4122C and located between the surrounding wall 4122B and the central part 4122C, a collection chamber is formed between the surrounding wall 4122B and the central part 4122C, and the collection port 401 is provided in the collection chamber. Therefore, after the second puncturing assembly 411 pierces the barrier, the liquid can be sucked reversely into the blood collection device 400 more quickly and enters the starting device 200 through the first fluid channel 4021 and the second fluid channel 4022.

The starting device 200 in this embodiment also includes a negative pressure tube 210, which has a pressure lower than atmospheric pressure. When the starting device 200 is inserted into the transmission device 300, the negative pressure tube 210 is in communication with the collection port 401 through the second fluid channel 4022. Through the negative pressure suction inside the negative pressure tube 210, the liquid inside the barrier can be sucked reversely into the collection device 400 via the collection port 401, and then flow into the starting device 200 via the second fluid channel 4022 to achieve liquid collection.

Optionally, in other embodiments, other structures that can achieve negative pressure suction can also be configured to replace the negative pressure tube 210 in this embodiment.

### Embodiment 14

As shown in Figures 10 and 15, based on Embodiment 13, this embodiment provides a specific structure of the second puncturing assembly 411. The second puncturing assembly 411 includes at least one microneedle 411A, which can be a solid puncturing needle, configured to pierce the barrier. The bottom wall 4122A is perforated with a through hole(s) 4122D, which corresponds to the at least one microneedle 411A one by one, opposite to the collection port 401 (in Figure 15, the through hole 4122D and the collection port 401 point to the same position).

The second puncturing assembly 411 is movable relative to the blood collection assembly 412 and is configured to pierce the barrier through the collection port 401. That is, when the starting device 200 is inserted into the housing 100, the driving assembly 320 can drive the microneedle 411A to penetrate through the through hole 4122D and pierce the barrier.

### Embodiment 15

As shown in Figure 15, in order to ensure the collection effect, in this embodiment, the end of the second fluid channel 4022 away from the negative pressure tube 210 extends into an accommodation space and is in communication with the collection port 401. The other end of the second fluid channel 4022 extends into the starting device 200. When the starting device 200 is inserted into the transmission device 300, the starting device 200 is in communication with the accommodation space and the collection port 401 through the second fluid channel 4022. Through the negative pressure suction inside the negative pressure tube 210, the liquid inside the barrier can be sucked reversely into the accommodation space via the collection port 401, and then flow into the starting device 200 via the second fluid channel 4022 to achieve liquid collection.

### Embodiment 16

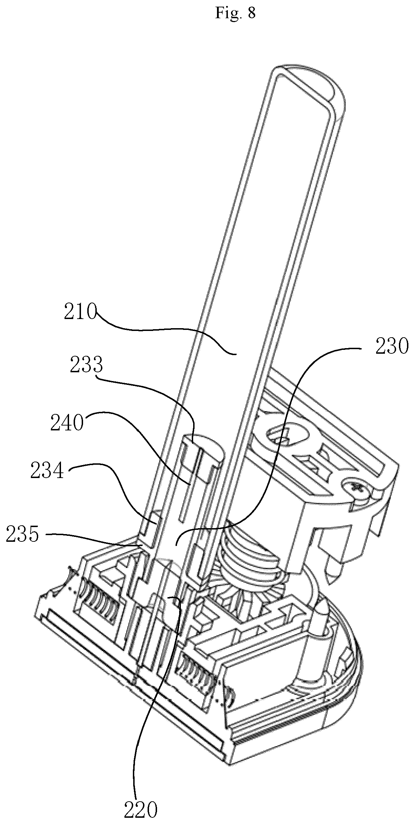

As shown in Figure 15, this embodiment provides a more specific solution for the structure of the blood collection assembly 412 based on Embodiment 15. In this solution, the blood collection assembly 412 includes a second base plate 412A and a first base plate 412B which are stacked along the movement direction of the second puncturing assembly 411. That is, the blood collection assembly 412 is a double walled structure, and the contact face 4121 is located on the side of the second base plate 412A away from the first base plate 412B. As shown in Figure 8, the second base plate 412A is penetrated to form a penetrated hole, and the inner wall of the penetrated hole is the surrounding wall 4122B. That is, a recess 4122 is provided on the blood collection assembly 412, the side wall of the recess 4122 is the surrounding wall 4122B, the bottom wall 4122A is located at the side of the first base plate 412B close to the second base plate 412A, and the central part 4122C is formed by extending from the first base plate 412B towards the second base plate 412A and is located within the penetrated hole and spaced apart from the surrounding wall.

Optionally, the blood collection assembly 412 includes an elastic seal (not shown), a first base plate 412B and a second base plate 412A, which are stacked along the first direction. The elastic seal (not shown) is provided between the first base plate 412B and the housing 100 for sealing, and the first base plate 412B and the second base plate 412A are provided outside the collection port 401 and connected to the housing 100. Optionally, the second base plate 412A is located on the outer side of the first base plate 412B. In this embodiment, the second fluid channel 4022 is provided on the first base plate 412B, and the end away from the central part 4122C penetrates the first base plate 412B and extends into the housing 100, and can be in communication with the starting device 200.

### Embodiment 17

As shown in Figure 10, the blood collection assembly 412 in this embodiment includes a contact face 4121 close to the barrier. When collecting is performed, the contact face 4121 is in close contact with the barrier, and the contact face 4121 is recessed in the direction away from the barrier to form a recess 4122. The recess 4122 includes a bottom wall 4122A opposite to the barrier and a surrounding wall 4122B which extends from the bottom wall 4122A towards the barrier and is connected between the contact face 4121 and the bottom wall 4122A. The bottom wall 4122A is provided with a central part 4122C which protrudes towards the barrier and is spaced apart from the surrounding wall 4122B, the collection port 401 is provided around an outer circumference of the central part 4122C and located between the surrounding wall 4122B and the central part 4122C, a collection chamber is formed between the surrounding wall 4122B and the central part 4122C, and the collection port 401 is provided in the collection chamber. Therefore, after the second puncturing assembly 411 pierces the barrier the liquid can be sucked reversely into the blood collection device more quickly and enters the collection device 400 through a flowing channel.

To ensure a closer contact with the barrier, the end face of the central part 4122C away from the bottom wall 4122A is coplanar with the contact face 4121. When collecting is performed, the end face of the central part 4122C away from the bottom wall 4122A is directly in close contact with the barrier, thereby making it easier for the second puncturing assembly 411 to puncture more stably.

### Embodiment 18

As shown in Figure 10, this embodiment is a further supplement to Embodiment 14. In this embodiment, the collection device 400 includes a second puncturing assembly 411 that is at least partially accommodated within the housing 100 and a blood collection assembly 412. The collection port 401 penetrates the blood collection assembly 412. The second puncturing assembly 411 is movable relative to the blood collection assembly 412 and is configured to pierce the barrier through the collection port 401. That is, when the starting device 200 is inserted into the housing 100, the driving assembly 320 can drive the second puncturing assembly 411 to penetrate through the collection port 401 and pierce the barrier 500. The blood collection assembly 412 includes a contact face 4121 close to the barrier 500. When collecting is performed, the contact face 4121 is in close contact with the barrier 500, and the contact face 4121 is recessed in the direction away from the barrier 500 to form a recess 4122. The recess 4122 includes a bottom wall 4122A opposite to the barrier and a surrounding wall 4122B which extends from the bottom wall 4122A towards the barrier and is connected between the contact face 4121 and the bottom wall 4122A.

The bottom wall 4122A is provided with a central part 4122C which protrudes towards the barrier and is spaced apart from the surrounding wall 4122B, the collection port 401 is provided around an outer circumference of the central part 4122C and located between the surrounding wall 4122B and the central part 4122C, a collection chamber is formed between the surrounding wall 4122B and the central part 4122C, and the collection port 401 is provided in the collection chamber. Therefore, after the second puncturing assembly 411 pierces the barrier, the liquid can be sucked reversely into the blood collection device more quickly and enters the collection device 400 through a flowing channel.

The starting device 200 in this embodiment also includes a negative pressure tube 210, which has a pressure lower than atmospheric pressure. When the starting device 200 is inserted into the transmission device 300, the negative pressure tube 210 is in communication with the collection port 401 through the second fluid channel 4022. Through the negative pressure suction inside the negative pressure tube 210, the liquid inside the barrier can be sucked reversely into the collection device 400 via the collection port 401, and then flow into the starting device 200 via the second fluid channel 4022 to achieve liquid collection.

The collection device 400 in this embodiment also includes a second puncturable seal 413 which is provided at the collection port 401 and located within the housing 100. The second puncturable seal 413 is connected to the second puncturing assembly 411, and the second puncturing assembly 411 extends through the second puncturable seal 413 and extends out of the housing under the action of the driving assembly. The second puncturable seal can move and deform with the second puncturing assembly 411 during the collection process.

Furthermore, the second puncturable seal 413 includes a first sealing part 413A fixed to the second puncturing assembly 411, a second sealing part 413C surrounding the first sealing part 413A, and an elastic deformation part 413B connected between the first sealing part 413A and the second sealing part 413C. The second puncturing assembly 411 extends through the first sealing part 413A towards the blood collection assembly 412, and the orthogonal projection of the second puncturable seal 413 onto the blood collection assembly 412 covers the whole collection port 401. The elastic deformation part 413B can undergo deformation.

Optionally, in other embodiments, the elastic deformation part 413B presents a shape that gradually thickens towards the blood collection assembly 412, and the second sealing part 413C is configured to extend towards the outside of the elastic deformation part 413B.

### Embodiment 19

As shown in Figures 10, 13, 14, 20 and 21, this embodiment is a further improvement of Embodiment 18. In this embodiment, the elastic deformation part 413B and the first sealing part 413A move with the second puncturing assembly 411. When the starting device 200 is inserted into the locking assembly 310, the locking assembly 310 releases the movement restriction on the driving assembly 320, so that the driving assembly 320 actuates the second puncturing assembly 411 to extend out of the housing 100 through the collection port 401 and pierce the barrier. During this process, the first sealing part 413A will move synchronously with the second puncturing assembly 411, and the elastic deformation part 413B will be compressed and deformed, and as it moves, when the liquid inside the barrier enters the collection device 400 through the collection port 401, under the action of atmospheric pressure, the elastic deformation part 413B will slide relative to the microneedle 411A towards the collection port 401 and ultimately press the through holes 4122D tightly, which prevents the liquid from entering the accommodation space inside 413B, and the liquid can only flow into the starting device 200 through the second fluid channel 4022. The starting device 200 can store the collected liquid in a timely manner, and when the starting device 200 is removed, the transfer of the liquid stored inside the starting device 200 can be achieved.

Furthermore, as shown in Figure 13, the second puncturing assembly 411 in this embodiment also includes a needle holder 411B associated with the transmission device 300, and the microneedle 411A is fixed to the needle holder 411B. The first sealing part 413A is fixed to the needle holder 411B, and the microneedle 411A penetrates the first sealing part 413A in advance and extends in the direction away from the needle holder 411B during assembly. Optionally, the first sealing part 413A can also be spaced apart from the second puncturing assembly 411, and the microneedle 411A, when performing the blood collection, pierces the first sealing part 413A and extends out of the housing.

Optionally, the needle holder 411B is cylindrical in shape and is provided with multiple mounting tooth holders around it. Each mounting tooth holder is correspondingly provided with a microneedle 411A, and the position limiting part 3121B can abut the other end of the needle holder 411B and limit the movement of the needle holder 411B. When the starting device 200 is inserted into the housing 100, the position limiting part 3121B can disengage from the needle holder 411B, thereby releasing the position limiting effect on the needle holder 411B, and at this time, under the action of the driving shaft 321 and the driving elastic piece 322, the driving shaft 321 will drive the needle holder 411B to move along the first direction, thereby causing the microneedle 411A to move along the first direction and penetrate the housing 100 through the collection port 401, and pierce the barrier 500.

### Embodiment 20

As shown in Figure 10, this embodiment is a further supplement to Embodiment 14. In this embodiment, the collection device 400 includes a second puncturing assembly 411 that is at least partially accommodated within the housing 100 and a blood collection assembly 412. The second puncturing assembly 411 is configured to pierce the barrier, and the collection port 401 penetrates the blood collection assembly 412; the second puncturing assembly 411 is movable relative to the blood collection assembly 412 and is configured to pierce the barrier through the collection port 401. That is, when the starting device 200 is inserted into the housing 100, the driving assembly 320 can drive the second puncturing assembly 411 to penetrate through the collection port 401 and pierce the barrier.

The blood collection assembly 412 includes a contact face 4121 close to the barrier. When collecting is performed, the contact face 4121 is in close contact with the barrier, and the contact face 4121 is recessed in the direction away from the barrier to form a recess 4122. The recess 4122 includes a bottom wall 4122A opposite to the barrier and a surrounding wall 4122B which extends from the bottom wall 4122A towards the barrier and is connected between the contact face 4121 and the bottom wall 4122A.

The bottom wall 4122A is provided with a central part 4122C which protrudes towards the barrier and is spaced apart from the surrounding wall 4122B, the collection port 401 is provided around an outer circumference of the central part 4122C and located between the surrounding wall 4122B and the central part 4122C, a collection chamber is formed between the surrounding wall 4122B and the central part 4122C, and the collection port 401 is provided in the collection chamber. Therefore, after the second puncturing assembly 411 pierces the barrier, the liquid can be sucked reversely into the blood collection device more quickly and enters the collection device 400 through a flowing channel, which also reduces injury area and pain.

As shown in Figures 9 and 18, the starting device 200 in this embodiment includes a negative pressure tube 210, which has a pressure lower than atmospheric pressure. When the starting device 200 is inserted into the transmission device 300, the negative pressure tube 210 is communicated with the collection port 401 through the second fluid channel 4022. Through the negative pressure suction inside the negative pressure tube 210, the liquid inside the barrier can be sucked reversely into the collection device 400 via the collection port 401, and then flow into the starting device 200 via the second fluid channel 4022 to achieve liquid collection.

In addition, the starting device 200 also includes a connector 220 that is detachably connected to the locking assembly 310, and a storage tube 230 that extends from the connector 220. The negative pressure tube 210 is in communication with the storage tube 230, and the storage tube 230 is located between the connector 220 and the negative pressure tube 210. The storage tube 230 is in communication with the second fluid channel 4022 through the connector 220.

When the starting device 200 is inserted into the transmission device 300, the connector 220 will be inserted into the locking assembly 310. Through the negative pressure suction inside the negative pressure tube 210, the liquid inside the barrier can be sucked reversely into the collection device 400 via the collection port 401, and then flow into the connector 220 via the second fluid channel 4022, and further into the storage tube 230 to achieve liquid collection.

The storage tube 230 is located between the connector 220 and the negative pressure tube 210. The storage tube 230 is in communication with the second fluid channel 4022 through the connector 220. Under the negative pressure suction effect of the negative pressure tube 210, blood or other liquids can smoothly enter the storage tube 230. When the starting device 200 is removed, the connector 220 disengages from the locking assembly 310 and blocks the storage tube 230 at an installation port 402, thereby achieving the storage and transfer of blood or other liquids and facilitating further detection.

### Embodiment 21

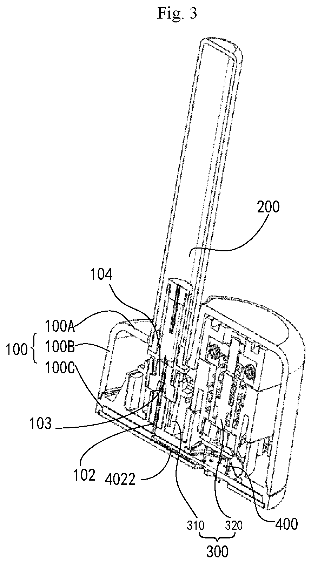

As shown in Figures 3 and 9, this embodiment provides a solution to achieve communication between the starting device 200 and the second fluid channel 4022. In this embodiment, the housing 100 has a first puncturing assembly 102, and one of the first puncturing assembly 102 and the connector 220 includes a first piercing piece 103 which has a hollow tubular shape with two ends open, and the other one includes a first puncturable seal 104 corresponding to the first piercing piece 103. The first piercing piece 103 is in communication with the second fluid channel 4022. When the first puncturing assembly 102 is connected to the connector 220, the first piercing piece 103 pierces the first puncturable seal 104 and establishes communication between the storage tube 230 and the second fluid channel 4022.

This embodiment provides two solutions. Solution I (as shown in Figures 1 and 7): the first piercing piece 103 is provided on the first puncturing assembly 102 inside the housing 100, and the first puncturable seal 104 is provided on the connector 220 of the starting device 200. Solution II (not shown in the drawings): the first puncturable seal 104 is provided on the first puncturing assembly 102 inside the housing 100, and the first piercing piece 103 is provided on the connector 220 of the starting device 200. Since the first piercing piece 103 in this solution is a hollow tubular structure with two ends open, both solutions can establish communication between the second fluid channel 4022 and the storage tube 230 when the connector 220 is connected to the first puncturing assembly 102.

### Embodiment 22

This embodiment is a further improvement based on Embodiment 21. In order to ensure the operational safety of the first piercing piece 103, as shown in Figures 3 and 9, in this embodiment, the first piercing piece 103 is connected to the first puncturing assembly 102 and located inside the housing 100, which provides higher safety. The locking assembly 310 is provided at the first puncturing assembly 102, and when the starting device 200 is inserted into the locking assembly 310, the connector 220 is docked with the first puncturing assembly 102, and the first piercing piece 103 pierces the first puncturable seal 104 and is in communication with the negative pressure tube 210, thereby establishing communication between the storage tube 230 and the second fluid channel 4022.

When the starting device 200 is inserted into the transmission device 300, under the negative pressure suction of the negative pressure tube 210, blood or other liquids can smoothly enter the storage tube 230. When the starting device 200 is removed, the connector 220 disengages from the first puncturing assembly 102 and blocks the storage tube 230 at an installation port 402, thereby achieving the storage and transfer of blood or other liquids and facilitating further detection.

A capillary tube 240 establishes communication between the negative pressure tube 210 and the storage tube 230, which realizes the transmission of negative pressure and the limitation and indication of the liquid volume. When the liquid enters the storage tube 230, as the liquid amount increases, the liquid level contacts the capillary tube, the liquid will not continue to fill the storage tube 230, but will overflow from the capillary tube and flow into the negative pressure tube 210, thereby achieving the liquid amount limitation and the indication. The capillary tube is made of a transparent material, generally a hydrophobic plastic material such as PC or PET.

### Embodiment 23

As shown in Figure 18, on the premise of adding a capillary tube 240 in Embodiment 22, this embodiment provides a specific setting method for the capillary tube 240. In this embodiment, the storage tube 230 includes a first open end 231 connected to the connector 220, and a second open end 232 opposite to the first open end 231. A first seal 233 is fitted inside the second open end 232, and the capillary tube 240 penetrates and is fixed to the first seal 233 and extends into the storage tube 230 through the second open end 232. The storage tube 230 is in communication with the negative pressure tube 210 through the capillary tube 240.

### Embodiment 24

As shown in Figure 18, this embodiment provides a preferred structure for the negative pressure tube 210. The negative pressure tube 210 is coaxially sleeved outside the storage tube 230, and the starting device 200 further includes a second seal 234 sandwiched between an outer wall of the storage tube 230 and an inner wall of the negative pressure tube 210, so as to form a vacuum space between the inner wall of the negative pressure tube 210 and the outer wall of the storage tube 230. The vacuum space is in communication with the storage tube 230 through the capillary tube 240. The setting of the first seal 233 and the second seal 234 can ensure that the negative pressure tube 210 and the storage tube 230 can only be in communication through the capillary tube 240, ensuring the sealing performance of the apparatus.

### Embodiment 25

As shown in Figure 18, in order to facilitate the connection between the storage tube 230 and the negative pressure tube 210, in this embodiment, a positioning part 235 protruding from the outer wall of the storage tube 230 is provided, and the positioning part 235 is configured to position the installation position of the negative pressure tube 210 relative to the storage tube 230. Specifically, the end of the negative pressure tube 210 close to the connector 220 abuts the side of the positioning part 235 away from the first open end 231, part of the second seal 234 is sandwiched between the positioning part 235 and the negative pressure tube 210, and another part of the second seal 234 is sandwiched between the inner wall of the negative pressure tube 210 and the outer wall of the storage tube 230 to avoid communication between the negative pressure tube 210 and the outside.

### Embodiment 26

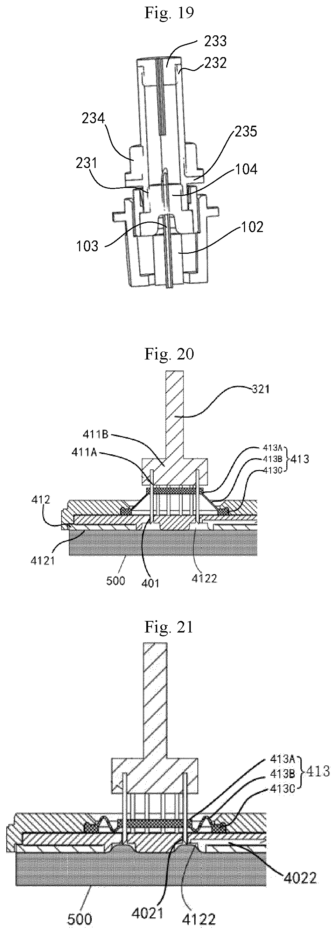

Furthermore, as shown in Figures 18 and 19, the connector 220 includes a first puncturable seal 104 that seals the first open end 231. The first piercing piece 103 is provided on the first puncturing assembly 102 inside the housing 100. When the starting device 200 is inserted into the housing 100, the connector 220 abuts the first puncturing assembly 102, and the first piercing piece 103 will penetrate the first puncturable seal 104, thereby establishing communication between the storage tube 230 and the second fluid channel 4022, and then under the action of the negative pressure tube 210, the liquid collected in the collection device 400 enters the storage tube 230 through the second fluid channel 4022 for storage. When the starting device 200 is removed, the first puncturable seal 104 will automatically block the puncturing port to prevent liquid leakage.

### Embodiment 27

This embodiment is a further improvement based on Embodiment 26. As shown in Figures 18 and 19, in this embodiment, the first puncturable seal 104 includes a fixed part 220A which is located outside the first open end 231 and has a size larger than the diameter of the storage tube 230; and a punctured part 220B extending from the fixed part 220A into the installation port 402 and matching the diameter of the storage tube 230. The fixed part 220A is configured to fix the entire first puncturable seal 104 at the first open end 231, so the size of the fixed part 220A needs to be larger than the diameter of the storage tube 230. The diameter of the punctured part 220B matches the diameter of the storage tube 230, and the punctured part 220B can be provided inside the storage tube 230. The fixed part 220A is fixed at the first open end 231. When the starting device 200 is inserted into the housing 100, the connector 220 abuts the first puncturing assembly 102, and then the first piercing piece 103 will penetrate the punctured part 220B, thereby establishing communication between the storage tube 230 and the second fluid channel 4022.

Optionally, in this embodiment, the connector 220 also includes a crown cover 221 that is sleeved on the outer circumference of the fixed part 220A and extends to the outer circumference of the storage tube 230. The setting of the crown cover 221 can provide a certain fixing effect on the fixed part 220A, avoiding the fixed part 220A from disengaging from the first open end 231.

Optionally, the fixed part 220A is provided with an unobstructed recess 220C extending to the punctured part 220B. When the connector 220 is connected to the first puncturing assembly 102, the first piercing piece 103 pierces the punctured part 220B through the unobstructed recess 220C and is in communication with the storage tube 230.

Optionally, the unobstructed recess 220C is a frustum-shaped recess with a gradually expanding diameter in the direction towards the first puncturing assembly 102, thereby facilitating the penetration of the first piercing piece 103.

### Embodiment 28

As shown in Figures 10 and 17, this embodiment provides an apparatus for collecting blood or other liquids, comprising a housing 100, a starting device 200, a transmission device 300, and a collection device 400. The housing 100 is provided with an installation port 402 and a collection port 401; the transmission device 300 is housed in the housing 100, and at least part of the starting device 200 is housed in the housing 100 and connected to the transmission device 300 through the installation port 402; at least part of the collection device 400 is housed in the housing 100, and the collection device 400 collects blood or other liquids through the collection port 401; the collection port 401 and the installation port 402 are not coaxially arranged.

In this case, the housing 100 and the starting device 200 of the apparatus are independently provided. The collection device 400 can only extend out of the housing 100 for collection when the starting device 200 is inserted into the housing 100, thereby avoiding accidental extension of the collection device 400 due to accidental contact and thus avoiding injuring a human body.

When in use, the starting device 200 is inserted into the installation port 402. During the insertion process, the starting device 200 can actuate the transmission motion, and then drive the collection device 400 to extend out of the housing through the transmission device 300 for the collection of blood or other liquids. In this solution, the collection port 401 and the installation port 402 are not coaxially arranged. If the collection port 401 and the installation port 402 are coaxially arranged, the entire apparatus will be too elongated in the direction along the collection channel, and this structural layout will be unreasonable. This solution provides a non-coaxial setting for them, preferably parallel setting, so as to flatten the overall apparatus and optimize the structure.

As shown in Figures 10, 11, and 17, in further embodiments, the housing 100 includes a lower housing 100C and an upper housing 100A, which are detachable. The transmission device 300, the collection device 400, and other components inside the housing 100 are first installed on the lower housing 100C, and then the upper housing 100A is snapped to complete the installation of the housing 100. Among them, the collection port 401 is provided at the lower housing 100C, while the installation port 402 is provided at the upper housing 100A.

Furthermore, a more detailed supplement is made to the relative position of the collection port 401 and the installation port 402 in the previous embodiment. The projection of the outer contour of the collection port 401 on the upper housing 100A does not overlap with the installation port 402, that is, the extension and retraction direction of the collection device 400 is not arranged to be coaxial with the insertion direction of the starting device 200, which also optimizes the structural layout of the apparatus.

Optionally, the starting device 200 in this embodiment includes a negative pressure tube 210, which has a pressure lower than atmospheric pressure. When the starting device 200 is inserted into the transmission device 300, the negative pressure tube 210 is in communication with a collection opening 501 through the second fluid channel 4022.

In addition, the starting device 200 also includes a connector 220 detachably connected to the transmission device 300 and a storage tube 230 extending from the connector 220. The negative pressure tube 210 is in communication with the storage tube 230, and the storage tube 230 is located between the connector 220 and the negative pressure tube 210. The storage tube 230 is in communication with the second fluid channel 4022 through the connector 220.

When the starting device 200 is inserted into the transmission device 300, through the negative pressure suction inside the negative pressure tube 210, the liquid inside the subject's skin can be sucked reversely into the collection device 400 via the collection opening 501, and then flows into the connector 220 via the second fluid channel 4022, and further into the storage tube 230 to achieve liquid collection.

As shown in Figure 3, the apparatus for collecting blood or other liquids in this solution includes a housing 100, a starting device 200, a transmission device 300, and a collection device 400. The transmission device 300 includes a locking assembly 310 and a driving assembly 320 which cooperate with each other. The locking assembly 310 can lock and unlock the driving assembly 320. The driving assembly 320 is connected to the collection device 400 and is configured to drive the collection device 400 to extend and retract. When the locking assembly 310 is in an unlocked state, the restriction on the driving assembly 320 is released, and the collection device 400 extends. The transmission device 300 and the collection device 400 are both provided within the housing 100. The starting device 200 is independent of the housing 100 and can be detachably connected to the locking assembly 310. After the starting device 200 is inserted, the locking assembly 310 releases the movement restriction on the driving assembly 320, so that the driving assembly 320 actuates the collection device 400 to extend out of the housing 100, and after the starting device 200 is removed, the locking assembly 310 restricts the movement of the driving assembly 320 to prevent the collection device 400 from extending, ensuring that the collection device 400 will not extend and injure a human body due to accidental contact, thereby improving the safety performance of the apparatus.

In this solution, the starting device 200 is detachably connected to the locking assembly 310. The collection device 400 can only extend when the starting device 200 is inserted into the locking assembly 310, which will not cause the collection device 400 to extend due to accidental contact or activation. This effectively solves the problem of blood collection apparatuses in the prior art that are prone to injury to a human body caused by extension of blood collection components due to accidental activation or contact.

Furthermore, as shown in Figure 4, the locking assembly 310 includes a pushing block 311 and sliding pieces 312. The pushing block 311 and the sliding pieces 312 are arranged to be relatively movable, and the movement of the pushing block 311 by itself can actuate the sliding piece 312 to move. This embodiment does not limit how the pushing block 311 drives the sliding pieces 312 to move. The pushing block 311 can be configured to directly contact and abut the sliding pieces 312, or indirectly drive the sliding pieces 312 to move through an intermediate.

The pushing block 311 is configured to move along a first direction under the action of the starting device 200 and drive the sliding pieces 312. The sliding pieces 312 are configured to move along a second direction under the action of the pushing block 311, to restrict the movement of the collection device 400 in the first direction and release the movement restriction on the collection device 400. The sliding pieces 312 can restrict the movement of the collection device 400 in the first direction, which is the direction in which the collection device 400 pierces a barrier. When the sliding pieces 312 are in a certain locking position, they can restrict the movement of the collection device 400 in the first direction, that is, limit the extension of the collection device 400. When the sliding pieces 312 move along the second direction under the action of the pushing block 311 to another unlocking position, they can release the movement restriction on the collection device 400 in the first direction, so that the collection device 400 can move along the first direction and extend out of the housing 100, and then complete the collection action.

In this solution, the sliding pieces 312 and the collection device 400 can be limited in position by a buckle-like structure, and when the sliding pieces 312 move along the second direction under the action of the pushing block 311 to another unlocking position, they separates at the buckle, releasing the position limitation on the driving assembly 320 and allowing it to drive the collection device 400 to extend.

When the starting device 200 is inserted in the pushing block 311 and the pushing block 311 is moved along the first direction, the pushing block 311 will drive the sliding piece 312 to move along the second direction to restrict the movement of the collection device 400 in the first direction and release the movement restriction on the collection device 400. In this solution, the first direction is not collinear with the second direction, which can make the layout of the entire apparatus more reasonable and avoid excessive elongation of the apparatus.

Optionally, the sliding piece 312 includes a first slider 3121 and a first deformable piece 3122 located between the first slider 3121 and the inner wall of the housing 100. The first deformable piece 3122 is connected to the first slider 3121 and is configured for reciprocating the first slider 3121 along the second direction. When the starting device 200 is inserted, it will actuate the first slider 3121 to squeeze and compress the first deformable piece 3122. When the starting device 200 is removed, the first deformable piece 3122 will return to its original state and actuate the first slider 3121 to reset, thereby re-limiting the position of the driving assembly 320.

Optionally, in this embodiment, a pair of first sliders 3121 are symmetrically arranged on the outer side of the pushing block 311, and the first elastic piece can be a spring. When the starting device 200 is inserted, the pushing block 311 will squeeze the pair of first sliders 3121 on its outer side in the second direction, and due to the compression of the spring being subject to pressure, at this time, the first sliders 3121 will move away from each other, thereby releasing the restriction on the driving assembly 320, and the driving assembly 320 will thereby drive the collection device 400 to extend out of the housing 100 along the first direction.

Furthermore, the sliding pieces 312 are symmetrically arranged on both sides of the collection device 400 along the second direction, which is different from only setting the sliding piece 312 on one side of the collection device 400. The sliding pieces 312 are symmetrically arranged on both sides of the collection device 400, such that the structure is more stable and the internal layout of the apparatus is more symmetrical and reasonable. The pushing block 311 is arranged between two sliding pieces 312 and pushes them to move in opposite directions, this layout is more reasonable. In addition, the pushing block 311 actuates the sliding pieces 312 to move synchronously towards opposite sides, which is more stable than single-side movement.

Furthermore, a more specific definition is made on the structure of the first slider 3121. The first slider 3121 has a body part 3121A and a position limiting part 3121B. The body part 3121A is connected to the first deformable piece 3122, and the position limiting part 3121B protrudes from the body part 3121A along the second direction. The position limiting part 3121B is configured to at least partially stop the collection device 400 in the first direction after the starting device 200 is removed, and release the stop on the collection device 400 after the starting device 200 is inserted. The position limiting effect on the collection device 400 is achieved through the position limiting part 3121B. Specifically, when the starting device 200 is not inserted, the collection device 400 is in a retracted state, and when the starting device 200 is inserted, the position limiting part 3121B releases the position limiting effect on the collection device 400, allowing the collection device 400 to extend.

Optionally, the position limiting part 3121B is an inverted triangular block. Before the starting device 200 is inserted, an upper part of the inverted triangular block abuts the driving assembly 320, thereby preventing the driving assembly 320 from driving the collection device 400 to extend; after the starting device 200 is inserted, the inverted triangular block will separate from the collection device 400, thereby releasing the restriction on the driving assembly 320, and thus allowing the driving assembly 320 to drive the collection device 400 to extend along the first direction and pierce the barrier.

Furthermore, as shown in Figure 4, this embodiment provides a more specific definition on the structure of the pushing block 311. The pushing block 311 has an inserting-connection part 3111 and squeezing parts 3112. The inserting-connection part 3111 is configured to form an inserting connection with the starting device 200, and the inserting-connection part 3111 can be a groove-shaped structure that can accommodate the starting device 200. The squeezing parts 3112 protrude from the outer side of the inserting-connection part 3111 and are configured to squeeze the sliding pieces 312 in the second direction. When the starting device 200 is inserted between two pushing blocks 311, the squeezing parts 3112 will squeeze the two sliding pieces 312 in the second direction to make them move away from each other, thereby causing the position limiting part 3121B to disengage from the collection device 400 and release the position limiting effect on the driving assembly 320.

Optionally, the cross-sectional area of the squeezing part 3112 gradually decreases along the insertion direction of the starting device 200, thereby facilitating the insertion of the starting device 200.

In this embodiment, the squeezing part 3112 is narrow at a lower end and wide at an upper end, wherein the narrower lower end facilitates insertion into the inserting-connection part 3111, and the wider upper end is configured for squeezing the sliding piece 312. Optionally, the squeezing part 3112 is an inverted right angled triangle structure, and its oblique edge squeezes the inserting-connection part 3111, and moves the inserting-connection part 3111, thereby pushing the sliding piece 312.

As shown in Figures 4 and 9, this embodiment further supplements the structure of the housing 100, which is provided with a guiding column 101. The inserting-connection part 3111 includes an inserting-connection section 3111A and a guiding section 3111B. The inserting-connection section 3111A is provided with an inserting-connection groove 3111C, and the guiding section 3111B is sleeved on the guiding column 101 to facilitate the installation of the guiding section 3111B and avoid errors that may affect accuracy. The squeezing part 3112 is provided on the guiding section 3111B, and specifically, it can be understood as the squeezing part 3112 being connected to the guiding section 3111B and integrally formed on an outside of the guiding section 3111B for squeezing the sliding piece 312. When the starting device 200 is inserted, the squeezing part 3112 outside the guiding section 3111B squeezes the sliding piece 312 in the second direction, causing the position limiting part 3121B to release its position limiting effect on the driving assembly 320, thereby allowing the collection device 400 to extend.

Furthermore, as shown in Figure 12, a further improvement has been made to the sliding piece 312. The sliding piece 312 is provided with a positioning portion 3121C corresponding to the squeezing parts 3112. The positioning portion 3121C is provided with accommodation parts 3121D that can accommodate the squeezing parts 3112, which are configured to limit the movement of the squeezing parts 3112. When the starting device 200 is inserted, the squeezing part 3112 squeezes the sliding piece 312. When the squeezing part 3112 moves to the lowest point, the squeezing part 3112 can enter the accommodation part 3121D and abut the positioning portion 3121C.

Furthermore, the driving assembly 320 is located between the collection device 400 and the inner wall of the housing 100, and is configured to drive the collection device 400 to extend from or retract into the housing 100. Specifically, when the starting device 200 is inserted into the housing 100, the sliding pieces 312 are squeezed to move away from each other, and at this time, the position limiting part 3121B on the sliding pieces 312 can release the movement restriction on the driving assembly 320, and thus the driving assembly 320 can drive the collection device 400 to extend. The driving assembly 320 includes a driving shaft 321 connected to the collection device 400 and a driving elastic piece 322 sleeved outside the driving shaft 321. When the sliding pieces 312 move to a state where the position limiting part 3121B disengages from the driving assembly 320, the sliding pieces 312 release the movement restriction on the collection device 400, and the driving elastic piece 322 pushes the driving shaft 321 along the first direction and actuates the collection device 400 to move towards outside of the housing 100 to a collection position. In this embodiment, the first direction in which the collection device 400 extends is parallel to the direction in which the starting device 200 is inserted.

Furthermore, as shown in Figure 10, in this embodiment, the driving assembly 320 includes not only the driving shaft 321 connected to the collection device 400 and the driving elastic piece 322 sleeved outside the driving shaft 321, but also a reset mechanism 323. After the collection device 400 moves to the collection position, the reset mechanism 323 pushes the collection device 400 to move towards inside of the housing 100. The reset mechanism 323 is separated from the driving elastic piece 322, that is, the driving assembly 320 can drive the collection device 400 to extend for collecting blood or other liquids, and can also drive the collection device 400 to retract after the collection is completed, so as to ensure sampling safety.

Optionally, the reset mechanism 323 includes a shaft sleeve 3231 sleeved outside the driving shaft 321 and a reset elastic piece 3232 connected to the shaft sleeve 3231. The driving shaft 321 is configured to pass through the shaft sleeve 3231 along the first direction and has a protrusion 321A protruding outward, that is, the driving shaft 321 is provided inside the shaft sleeve 3231, and the shaft sleeve 3231 is provided inside the reset elastic piece 3232. After the collection device 400 moves to the collection position, the reset elastic piece 3232 pushes the shaft sleeve 3231 and actuates the driving shaft 321 and the collection device 400 to move towards inside of the housing 100 through the protrusion 321A.

Optionally, in other preferred embodiments, a bottom of the shaft sleeve 3231 is provided an abutting part 3233 which extends inward and can abut the protrusion 321A. When the reset elastic piece 3232 tends to reset and rebounds, the abutting part 3233 will abut the protrusion 321A and actuate the protrusion 321A to move in the direction in which the reset elastic piece 3232 rebounds, that is, actuate the driving shaft 321 to move in the direction in which the reset elastic piece 3232 rebounds, thereby moving the collection device 400 towards inside of the housing 100.

Optionally, the end of the reset elastic piece 3232 away from the collection device 400 is fixed to the shaft sleeve 3231, and the end of the reset elastic piece 3232 close to the collection device 400 is configured to be movable relative to the shaft sleeve 3231 along the first direction relative to the housing 100.

Furthermore, an upper half of the shaft sleeve 3231 is provided with a fixed part 220A protruding outward. One end of the reset elastic piece 3232 is fixed inside the fixed part 220A, and the other end is configured to be movable relative to the shaft sleeve 3231 along the first direction. When the other end moves in the direction opposite to the first direction, it can actuate the shaft sleeve 3231 to move in the same direction, and then actuate the driving shaft 321 to move towards inside of the housing 100 through the protrusion 321A, so that the collection device 400 is retracted into the housing 100.

Furthermore, as shown in Figure 10, this embodiment proposes a better choice for the driving elastic piece 322 and the reset elastic piece 3232. Both the driving elastic piece 322 and the reset elastic piece 3232 are precompressed springs, which can increase the load capacity of the springs. The reset elastic piece 3232 is sleeved on an outer circumference of the shaft sleeve 3231. The elastic coefficient of the driving elastic piece 322 is greater than that of the reset elastic piece 3232, so that, after the position limiting part 3121B releases the restriction on the collection device 400, the elasticity of the driving elastic piece 322 can resist the elasticity of the reset elastic piece 3232, and then the collection device 400 extends.

Furthermore, in order to better understand how the driving elastic piece 322 works, this embodiment provides a feasible solution. In this embodiment, the driving assembly 320 also includes a top position-limiting mechanism 324 located at the end of the driving elastic piece 322 away from the collection device 400. The top position-limiting mechanism 324 is configured to precompress the driving elastic piece 322, that is, the driving elastic piece 322 is located between the protrusion 321A on the side wall of the driving shaft 321 and the top position-limiting mechanism 324. When the position limiting part 3121B releases the position limiting effect on the collection device 400, the driving shaft 321 will move along the first direction under the action of the driving elastic piece 322 and drive the collection device 400 to extend out of the housing 100. When the top position-limiting mechanism 324 releases the precompressing effect on the driving elastic piece 322, the driving elastic piece 322 will move towards the top position-limiting mechanism 324.

Furthermore, this embodiment provides a specific implementation for the cooperation between the top position-limiting mechanism 324 and the driving shaft 321, which can be combined with other embodiments. In this embodiment, the top position-limiting mechanism 324 is provided with an unobstructed part 3241 corresponding to the driving shaft 321. The end of the driving shaft 321 away from the collection device 400 can pass through the unobstructed part 3241, and the end of the driving shaft 321 away from the collection device 400 extends outside the top position-limiting mechanism 324 through the unobstructed part 3241 and is spaced apart from the top position-limiting mechanism 324.

In some embodiments, a more detailed description of the specific layout of the top position-limiting mechanism 324 is provided. The top position-limiting mechanism 324 includes second sliders 3242 located on opposite sides of the driving shaft 321 and spaced apart from each other, as well as precompressed position-limiting elastic pieces 3243 connected between the two second sliders 3242 that are arranged to be opposite. The top sliders and the precompressed position-limiting elastic pieces 3243 enclose the unobstructed part 3241. A position-limiting groove 32421 is provided on the side of the second slider 3242 close to the collection device 400, which is configured to form a snapping connection with the shaft sleeve 3231. Specifically, the side of the shaft sleeve 3231 away from the collection device 400 forms the snapping connection with the position-limiting groove 32421 to limit the movement of the two second sliders 3242. When the shaft sleeve 3231 moves along the first direction under the action of the driving elastic piece 322, the shaft sleeve 3231 will disengage from the position-limiting groove 32421, thereby releasing the position limiting effect on the two second sliders 3242.

When the collection device 400 moves to the collection position, the driving elastic piece 322 drives the driving shaft 321 to actuate the shaft sleeve 3231 to move and disengage from the position-limiting groove 32421. The precompressed position-limiting elastic piece 3243 drives the two second sliders 3242 to separate, and then the gap of the unobstructed part 3241 increases. The end of the driving elastic piece 322 away from the collection device 400 is released through the gap generated by the separation between the second sliders 3242, and at this time, the reset elastic piece 3232 pushes the shaft sleeve 3231 and actuates the driving shaft 321 and the collection device 400 to move towards inside of the housing 100 through the protrusion 321A, thereby resetting the collection device 400.

Furthermore, in this embodiment, a structure different from the top position-limiting mechanism 324 in Embodiment 9 is provided. The top position-limiting mechanism 324 includes second sliders 3242 located on opposite sides of the driving shaft 321 and a pre-stretched position-limiting elastic piece connected between the slider and the housing 100, wherein an unobstructed part 3241 is formed between the second sliders 3242.

In the initial state, the pre-stretched position-limiting elastic piece is in an extended and stretched state. A position-limiting groove 32421 is provided on the side of the second slider 3242 close to the collection device 400, which is configured to form a snapping connection with the shaft sleeve 3231. Specifically, the side of the shaft sleeve 3231 away from the collection device 400 forms the snapping connection with the position-limiting groove 32421 to limit the movement of the two second sliders 3242. When the shaft sleeve 3231 moves along the first direction under the action of the driving elastic piece 322, the shaft sleeve 3231 will disengage from the position-limiting groove 32421, thereby releasing the position limiting effect on the two second sliders 3242.

When the collection device 400 moves to the collection position, the driving elastic piece 322 drives the driving shaft 321 to actuate the shaft sleeve 3231 to move and disengage from the position-limiting groove 32421. The pre-stretched position-limiting elastic piece drives the two second sliders 3242 to separate, and then the gap of the unobstructed part 3241 increases. The end of the driving elastic piece 322 away from the collection device 400 is released through the gap generated by the separation between the second sliders 3242, and at this time, the reset elastic piece 3232 pushes the shaft sleeve 3231 and actuates the driving shaft 321 and the collection device 400 to move towards inside of the housing 100 through the protrusion 321A, thereby resetting the collection device 400.

Furthermore, to ensure a more compact overall structure of the apparatus, in this embodiment, the first direction is configured to be perpendicular to the second direction, and components, such as the pushing block 311 and the sliding pieces 312, are symmetrically distributed inside the housing 100. The layout is more reasonable.

Optionally, the first direction is parallel to the direction in which the collection device 400 pierces the barrier, and the second direction is parallel to the tangent plane of the barrier surface, to ensure piercing the barrier orthogonally and thus ensure the collection effect.

Furthermore, this embodiment further improves how the collection device 400 achieves the collection function. Specifically, a collection port 401 is provided at the side of the housing 100 close to the collection device 400, and the transmission device 300 drives the collection device 400 along the first direction to extend out of the housing 100 through the collection port 401.

After the starting device 200 is inserted, the locking assembly 310 releases the movement restriction on the driving assembly 320, so that the driving assembly 320 actuates the collection device 400 to extend out of the housing 100 through the collection port 401, and after the starting device 200 is removed, the locking assembly 310 restricts the movement of the driving assembly 320, which makes it impossible for the collection device 400 to extend, ensuring that the collection device 400 will not extend and injure a human body due to accidental contact, thereby improving the safety performance of the apparatus.

When the starting device 200 is inserted into the housing 100, the sliding pieces 312 are squeezed to move away from each other, and at this time, the position limiting part 3121B releases the movement restriction on the driving assembly 320, and then the driving assembly 320 can drive the collection device 400 to extend from the collection port 401. The driving assembly 320 includes a driving shaft 321 connected to the collection device 400 and a driving elastic piece 322 sleeved outside the driving shaft 321. When the sliding piece 312 moves to a state where the position limiting part 3121B disengages from the driving assembly 320, the sliding pieces 312 release the movement restriction on the collection device 400, and the driving elastic piece 322 pushes the driving shaft 321 along the first direction and actuates the collection device 400 to move towards outside of the housing 100 to the collection position through the collection port 401. After piercing the barrier, the collection device enters the housing 100 through the collection port 401 again. In this embodiment, the first direction in which the collection device 400 extends is parallel to the direction in which the starting device 200 is inserted.

Furthermore, in order to ensure the collection effect and timely recover and store the collected blood or other liquids, it is necessary to transfer the liquid collected by the collection device 400 in a timely manner. To achieve this goal, this application provides a second fluid channel 4022 which is in communication with the collection port 401 in the housing 100. The flowing channel can be provided in a snake curved shape in the housing 100. The starting device 200 is in communication with the collection port 401 through the second fluid channel 4022. After the starting device 200 is inserted into the locking assembly 310, the locking assembly 310 releases the movement restriction on the driving assembly 320, so that the driving assembly 320 actuates the collection device 400 to extend out of the housing 100 through the collection port 401 and pierce the barrier. After the liquid inside the barrier enters the collection device 400 through the collection port 401, it will promptly flow into the starting device 200 by the second fluid channel 4022. The starting device 200 can store the collected liquid in a timely manner. When the starting device 200 is removed, the transfer of the liquid stored inside the starting device 200 can be achieved.

Optionally, in order to ensure smooth collection of liquid from the barrier, this embodiment provides a specific structure of the starting device 200. The starting device 200 includes a negative pressure tube 210, which has a pressure lower than atmospheric pressure. When the starting device 200 is inserted into the transmission device 300, the negative pressure tube 210 is in communication with the collection port 401 through the second fluid channel 4022. Through the negative pressure suction inside the negative pressure tube 210, the liquid inside the barrier can be sucked reversely into the collection device 400 via the collection port 401, and then flow into the starting device 200 via the second fluid channel 4022 to achieve liquid collection.

Furthermore, this embodiment provides a specific structure of the collection device 400. The collection device 400 includes a housing 100 with an accommodation space and a second puncturing assembly 411 which is at least partially accommodated within the accommodation space. The housing 100 includes a contact wall, and the collection port 401 penetrates the contact wall; the second puncturing assembly 411 is movable relative to the contact wall and is configured to pierce the barrier through the collection port 401. That is, when the starting device 200 is inserted into the housing 100, the driving assembly 320 can drive the second puncturing assembly 411 to penetrate through the collection port 401 and pierce the barrier.

The contact wall includes a contact face 4121 close to the barrier. When collecting is performed, the contact face 4121 is in close contact with the barrier, and the contact face 4121 is recessed in the direction away from the barrier to form a recess 4122. The recess 4122 includes a bottom wall 4122A opposite to the barrier and a surrounding wall 4122B which extends from the bottom wall 4122A towards the barrier and is connected between the contact face 4121 and the bottom wall 4122A.

The bottom wall 4122A is provided with a central part 4122C which protrudes towards the barrier and is spaced apart from the surrounding wall 4122B, the collection port 401 is provided around an outer circumference of the central part 4122C and located between the surrounding wall 4122B and the central part 4122C, a collection chamber is formed between the surrounding wall 4122B and the central part 4122C, and the collection port 401 is provided in the collection chamber. Therefore, after the second puncturing assembly 411 pierces the barrier, the liquid can be sucked reversely into the blood collection device more quickly and enters the collection device 400 through the flowing channel.

The starting device 200 in this embodiment also includes a negative pressure tube 210, which has a pressure lower than atmospheric pressure. When the starting device 200 is inserted into the transmission device 300, the negative pressure tube 210 is in communication with the collection port 401 through the second fluid channel 4022. Through the negative pressure suction inside the negative pressure tube 210, the liquid inside the barrier can be sucked reversely into the collection device 400 via the collection port 401, and then flow into the starting device 200 via the second fluid channel 4022 to achieve liquid collection.

Optionally, in other embodiments, other structures that can achieve negative pressure suction can also be configured to replace the negative pressure tube 210 in this embodiment.

Furthermore, this embodiment provides a specific structure of the second puncturing assembly 411. The second puncturing assembly 411 includes at least one microneedle 411A, which can be a solid puncturing needle, configured to pierce the barrier. The bottom wall 4122A is perforated with a through hole(s) 4122D, which corresponds to the at least one microneedle 411A one by one, opposite to the collection port 401.

The second puncturing assembly 411 is movable relative to the contact wall and is configured to pierce the barrier through the collection port 401. That is, when the starting device 200 is inserted into the housing 100, the driving assembly 320 can drive the microneedle 411A to penetrate through the through hole 4122D and pierce the barrier.

Furthermore, in order to ensure the collection effect, in this embodiment, the end of the second fluid channel 4022 away from the negative pressure tube 210 extends into an accommodation space and is in communication with the collection port 401. The other end of the second fluid channel 4022 extends into the starting device 200. When the starting device 200 is inserted into the transmission device 300, the starting device 200 is in communication with the accommodation space and the collection port 401 through the second fluid channel 4022. Through the negative pressure suction inside the negative pressure tube 210, the liquid inside the barrier can be sucked reversely into the accommodation space via the collection port 401, and then flow into the starting device 200 via the second fluid channel 4022 to achieve liquid collection.

Furthermore, a more specific solution is provided for the structure of the contact wall. In this solution, the contact wall includes a first contact wall and a second contact wall which are stacked along the movement direction of the second puncturing assembly 411, that is, the contact wall is a double walled structure. The contact face 4121 is located on the side of the first contact wall away from the second contact wall, and the first contact wall is penetrated to form a surrounding wall 4122B. That is, a recess 4122 is provided on the first contact wall, and the side wall of the recess 4122 is the surrounding wall 4122B. The bottom wall 4122A is located on the side of the second contact wall close to the first contact wall, and the central part 4122C is formed by extending from the second contact wall towards the first contact wall.

Optionally, the first contact wall is located on the outer side of the second contact wall. In this embodiment, the second fluid channel 4022 is provided on the second contact wall, and the end away from the central part 4122C penetrates the second contact wall and extends into the housing 100, and can be in communication with the starting device 200.

Furthermore, the contact wall in this embodiment includes a contact face 4121 close to the barrier. When collecting is performed, the contact face 4121 is in close contact with the barrier, and the contact face 4121 is recessed in the direction away from the barrier to form a recess 4122. The recess 4122 includes a bottom wall 4122A opposite to the barrier and a surrounding wall 4122B which extends from the bottom wall 4122A towards the barrier and is connected between the contact face 4121 and the bottom wall 4122A. The bottom wall 4122A is provided with a central part 4122C which protrudes towards the barrier and is spaced apart from the surrounding wall 4122B, the collection port 401 is provided around an outer circumference of the central part 4122C and located between the surrounding wall 4122B and the central part 4122C, a collection chamber is formed between the surrounding wall 4122B and the central part 4122C, and the collection port 401 is provided in the collection chamber. Therefore, after the second puncturing assembly 411 pierces the barrier, the liquid can be sucked reversely into the blood collection device more quickly and enters the collection device 400 through a flowing channel.

To ensure a closer contact with the barrier, the end face of the central part 4122C away from the bottom wall 4122A is coplanar with the contact face 4121. When collecting is performed, the end face of the central part 4122C away from the bottom wall 4122A is directly in close contact with the barrier, thereby making it easier for the second puncturing assembly 411 to puncture more stably.

Furthermore, in this embodiment, the collection device 400 includes a housing 100 with an accommodation space and a second puncturing assembly 411 which is at least partially accommodated within the accommodation space. The housing 100 includes a contact wall, and the collection port 401 penetrates the contact wall; the second puncturing assembly 411 is movable relative to the contact wall and is configured to pierce the barrier through the collection port 401. That is, when the starting device 200 is inserted into the housing 100, the driving assembly 320 can drive the second puncturing assembly 411 to penetrate through the collection port 401 and pierce the barrier 500.

The contact wall includes a contact face 4121 close to the barrier. When collecting is performed, the contact face 4121 is in close contact with the barrier, and the contact face 4121 is recessed in the direction away from the barrier to form a recess 4122. The recess 4122 includes a bottom wall 4122A opposite to the barrier and a surrounding wall 4122B which extend from the bottom wall 4122A towards the barrier and is connected between the contact face 4121 and the bottom wall 4122A.

The bottom wall 4122A is provided with a central part 4122C which protrudes towards the barrier 500 and is spaced apart from the surrounding wall 4122B, the collection port 401 is provided around an outer circumference of the central part 4122C and located between the surrounding wall 4122B and the central part 4122C, a collection chamber is formed between the surrounding wall 4122B and the central part 4122C, and the collection port 401 is provided in the collection chamber. Therefore, after the second puncturing assembly 411 pierces the barrier, the liquid can be sucked reversely into the blood collection device more quickly and enters the collection device 400 through the flowing channel.

The starting device 200 in this embodiment also includes a negative pressure tube 210, which has a pressure lower than atmospheric pressure. When the starting device 200 is inserted into the transmission device 300, the negative pressure tube 210 is in communication with the collection port 401 through the second fluid channel 4022. Through the negative pressure suction inside the negative pressure tube 210, the liquid inside the barrier can be sucked reversely into the collection device 400 via the collection port 401, and then flow into the starting device 200 via the second fluid channel 4022 to achieve liquid collection.

The starting device 200 in this embodiment also includes a flexible seal that is configured to cover the side of the contact wall facing away from the contact face 4121 and connected between the second puncturing assembly 411 and the contact wall. The flexible seal can deform during the collection process.

The flexible seal includes a first sealing part 413A fixed to the puncturing assembly, a second sealing part 413C surrounding the first sealing part 413A, and a flexible sealing ring (an elastic deformation part 413B) connected between the central fixed part (a first sealing part 413A) and the edge fixed part (a second sealing part 413C). The second puncturing assembly 411 extends through the central fixed part towards the contact wall, and the orthogonal projection of the flexible seal onto the contact wall covers the whole collection port 401. The flexible sealing ring can deform.

Optionally, in other embodiments, the flexible sealing ring presents a shape that gradually thickens towards the contact wall, and the second sealing part 413C is configured to extend towards the outside of the flexible sealing ring.

Furthermore, in this embodiment, the flexible sealing ring and the central fixed part move with the second puncturing assembly 411. When the starting device 200 is inserted into the locking assembly 310, the locking assembly 310 releases the movement restriction on the driving assembly 320, so that the driving assembly 320 actuates the second puncturing assembly 411 to extend out of the housing 100 through the collection port 401 and pierce the barrier. During this process, the central fixed part will move synchronously with the second puncturing assembly 411, and the flexible sealing ring will be compressed and deformed, and as it moves, when the liquid inside the barrier enters the collection device 400 through the collection port 401, under the action of atmospheric pressure, the elastic deformation part 413B will slide relative to the microneedle 411A towards the collection port 401 and ultimately press the through holes 4122D tightly, which prevents the liquid from entering the accommodation space inside 413B, and the liquid can only flow into the starting device 200 through the second fluid channel 4022. The starting device 200 can store the collected liquid in a timely manner, and when the starting device 200 is removed, the transfer of the liquid stored inside the starting device 200 can be achieved.

Furthermore, the second puncturing assembly 411 in this embodiment also includes a needle holder 411B associated with the transmission device 300, and the microneedle 411A is fixed to the needle holder 411B.

Optionally, the needle holder 411B is cylindrical in shape and is provided with multiple mounting tooth holders around it. Each mounting tooth holder is correspondingly provided with a microneedle 411A, and the position limiting part 3121B can abut the other end of the needle holder 411B and limit the movement of the needle holder 411B. When the starting device 200 is inserted into the housing 100, the position limiting part 3121B can disengage from the needle holder 411B, thereby releasing the position limiting effect on the needle holder 411B, and at this time, under the action of the driving shaft 321 and the driving elastic piece 322, the driving shaft 321 will drive the needle holder 411B to move along the first direction, thereby causing the microneedle 411A to move along the first direction and penetrate the housing 100 through the through hole 4122D, and pierce the barrier.

Furthermore, in this embodiment, the collection device 400 includes a housing 100 with an accommodation space and a second puncturing assembly 411 which is at least partially accommodated within the accommodation space. The second puncturing assembly 411 is configured to pierce the barrier. The housing 100 includes a contact wall, and the collection port 401 penetrates the contact wall; the second puncturing assembly 411 is movable relative to the contact wall and is configured to pierce the barrier through the collection port 401. That is, when the starting device 200 is inserted into the housing 100, the driving assembly 320 can drive the second puncturing assembly 411 to penetrate through the collection port 401 and pierce the barrier.

The contact wall includes a contact face 4121 close to the barrier. When collecting is performed, the contact face 4121 is in close contact with the barrier, and the contact face 4121 is recessed in the direction away from the barrier to form a recess 4122. The recess 4122 includes a bottom wall 4122A opposite to the barrier and a surrounding wall 4122B which extends from the bottom wall 4122A towards the barrier and is connected between the contact face 4121 and the bottom wall 4122A.

The bottom wall 4122A is provided with a central part 4122C which protrudes towards the barrier and is spaced apart from the surrounding wall 4122B, the collection port 401 is provided around an outer circumference of the central part 4122C and located between the surrounding wall 4122B and the central part 4122C, a collection chamber is formed between the surrounding wall 4122B and the central part 4122C, and the collection port 401 is provided in the collection chamber. Therefore, after the second puncturing assembly 411 pierces the barrier, the liquid can be sucked reversely into the blood collection device more quickly and enters the collection device 400 through a flowing channel.

The starting device 200 in this embodiment includes a negative pressure tube 210, which has a pressure lower than atmospheric pressure. When the starting device 200 is inserted into the transmission device 300, the negative pressure tube 210 is communicated with the collection port 401 through the second fluid channel 4022. Through the negative pressure suction inside the negative pressure tube 210, the liquid inside the barrier can be sucked reversely into the collection device 400 via the collection port 401, and then flow into the starting device 200 via the second fluid channel 4022 to achieve liquid collection.

In addition, the starting device 200 also includes a connector 220 that is detachably connected to the locking assembly 310, and a storage tube 230 that extends from the connector 220. The negative pressure tube 210 is in communication with the storage tube 230, and the storage tube 230 is located between the connector 220 and the negative pressure tube 210. The storage tube 230 is in communication with the second fluid channel 4022 through the connector 220.

When the starting device 200 is inserted into the transmission device 300, the connector 220 will be inserted into the locking assembly 310. Through the negative pressure suction inside the negative pressure tube 210, the liquid inside the barrier can be sucked reversely into the collection device 400 via the collection port 401, and then flow into the connector 220 via the second fluid channel 4022, and further into the storage tube 230 to achieve liquid collection.

The storage tube 230 is located between the connector 220 and the negative pressure tube 210. The storage tube 230 is in communication with the second fluid channel 4022 through the connector 220. Under the negative pressure suction effect of the negative pressure tube 210, blood or other liquids can smoothly enter the storage tube 230. When the starting device 200 is removed, the connector 220 disengages from the locking assembly 310 and blocks the storage tube 230 at an installation port 402, thereby achieving the storage and transfer of blood or other liquids and facilitating further detection.

Furthermore, this embodiment provides a solution to achieve communication between the starting device 200 and the second fluid channel 4022. In this embodiment, the housing 100 has a mounting part, and one of the mounting part and the connector 220 includes a first piercing piece 103 which has a hollow tubular shape with two ends open, and the other one includes a pierceable seal corresponding to the first piercing piece 103. The first piercing piece 103 is in communication with the second fluid channel 4022. When the mounting part is connected to the connector 220, the first piercing piece 103 pierces the pierceable seal and establishes communication between the storage tube 230 and the second fluid channel 4022.

This embodiment provides two solutions. Solution I: the first piercing piece 103 is provided on the mounting part inside the housing 100, and the pierceable seal is provided on the connector 220 of the starting device 200. Solution II: the pierceable seal is provided on the mounting part inside the housing 100, and the first piercing piece 103 is provided on the connector 220 of the starting device 200. Since the first piercing piece 103 in this solution is a hollow tubular structure with two ends open, both solutions can establish communication between the second fluid channel 4022 and the storage tube 230 when the connector 220 is connected to the mounting part.

Furthermore, in order to ensure the operational safety of the first piercing piece 103, in this embodiment, the first piercing piece 103 is connected to the mounting part and located inside the housing 100, which provides higher safety. The locking assembly 310 is provided at the mounting part, and when the starting device 200 is inserted into the locking assembly 310, the connector 220 is docked with the mounting part, and the first piercing piece 103 pierces the pierceable seal and is in communication with the negative pressure tube 210, thereby establishing communication between the storage tube 230 and the second fluid channel 4022.

When the starting device 200 is inserted into the transmission device 300, under the negative pressure suction of the negative pressure tube 210, blood or other liquids can smoothly enter the storage tube 230. When the starting device 200 is removed, the connector 220 disengages from the mounting part and blocks the storage tube 230 at an installation port 402, thereby achieving the storage and transfer of blood or other liquids and facilitating further detection.

Furthermore, this embodiment provides a specific setting method for the capillary tube 240. In this embodiment, the storage tube 230 includes a first open end 231 connected to the connector 220, and a second open end 232 opposite to the first open end 231. A first seal 233 is fitted inside the second open end 232, and the capillary tube 240 penetrates and is fixed to the first seal 233 and extends into the storage tube 230 through the second open end 232. The storage tube 230 is in communication with the negative pressure tube 210 through the capillary tube 240.

Furthermore, this embodiment provides a preferred structure for the negative pressure tube 210. The negative pressure tube 210 is coaxially sleeved outside the storage tube 230, and the starting device 200 further includes a second seal 234 sandwiched between an outer wall of the storage tube 230 and an inner wall of the negative pressure tube 210, so as to form a vacuum space between the inner wall of the negative pressure tube 210 and the outer wall of the storage tube 230. The vacuum space is in communication with the storage tube 230 through the capillary tube 240. The setting of the first seal 233 and the second seal 234 can ensure that the negative pressure tube 210 and the storage tube 230 can only be in communication through the capillary tube 240, ensuring the sealing performance of the apparatus.

Furthermore, in order to facilitate the connection between the storage tube 230 and the negative pressure tube 210, in this embodiment, a positioning part 235 protruding from the outer wall of the storage tube 230 is provided, and the positioning part 235 is configured to position the installation position of the negative pressure tube 210 relative to the storage tube 230. Specifically, the end of the negative pressure tube 210 close to the connector 220 abuts the side of the positioning part 235 away from the first open end 231, part of the second seal 234 is sandwiched between the positioning part 235 and the negative pressure tube 210, and another part of the second seal 234 is sandwiched between the inner wall of the negative pressure tube 210 and the outer wall of the storage tube 230 to avoid communication between the negative pressure tube 210 and the outside.

Furthermore, the connector 220 includes a pierceable seal that seals the first open end 231. The first piercing piece 103 is provided on the mounting part inside the housing 100. When the starting device 200 is inserted into the housing 100, the connector 220 abuts the mounting part, the first piercing piece 103 will penetrate the pierceable seal, thereby establishing communication between the storage tube 230 and the second fluid channel 4022, and then under the action of the negative pressure tube 210, the liquid collected in the collection device 400 enters the storage tube 230 through the second fluid channel 4022 for storage. When the starting device 200 is removed, the pierceable seal will automatically block the puncturing port to prevent liquid leakage.

Furthermore, as shown in Figures 18 and 19, in this embodiment, the pierceable seal includes a fixed part 220A which is located outside the first open end 231 and has a size larger than the diameter of the storage tube 230; and a punctured part 220B extending from the fixed part 220A into the installation port 402 and matching the diameter of the storage tube 230. The fixed part 220A is configured to fix the entire pierceable seal at the first open end 231, so the size of the fixed part 220A needs to be larger than the diameter of the storage tube 230. The diameter of the punctured part 220B matches the diameter of the storage tube 230, and the punctured part 220B can be provided inside the storage tube 230. The fixed part 220A is fixed at the first open end 231. When the starting device 200 is inserted into the housing 100, the connector 220 abuts the mounting part, and then the first piercing piece 103 will penetrate the punctured part 220B, thereby establishing communication between the storage tube 230 and the second fluid channel 4022.

Optionally, in this embodiment, the connector 220 also includes a crown cover 221 that is sleeved on the outer circumference of the fixed part 220A and extends to the outer circumference of the storage tube 230. The setting of the crown cover 221 can provide a certain fixing effect on the fixed part 220A, avoiding the fixed part 220A from disengaging from the first open end 231.

Optionally, the fixed part 220A is provided with an unobstructed recess 220C extending to the punctured part 220B. When the connector 220 is connected to the mounting part, the first piercing piece 103 pierces the punctured part 220B through the unobstructed recess 220C and is in communication with the storage tube 230.

Optionally, the unobstructed recess 220C is a frustum-shaped recess 4122 with a gradually expanding diameter in the direction towards the mounting part, thereby facilitating the penetration of the first piercing piece 103.

### Embodiment 29

This embodiment provides a method for collecting blood or other liquids from a subject, comprising:
applying a housing 100 to a skin of a subject, so that a collection port 401 on the housing 100 are aligned with the corresponding collection site on the subject's skin orthogonally, wherein the housing 100 is provided with a collection device 400 therein, which is extendable and retractable relative to the housing 100, as well as a transmission device 300 associated with the collection device 400, wherein the collection device 400 is driven by the transmission device 300 to extend out of the housing 100 and pierce the subject's skin, and the structure of the transmission device 300 may be implemented with reference to relevant descriptions in other embodiments;
when in use, inserting a starting device 200 into the transmission device 300 inside the housing 100, to drive the transmission device 300 to move, the transmission device 300 further driving the collection device 400 to extend out of the housing 100 along a first direction to pierce the skin of the subject, wherein preferably the extension direction of the collection device 400 is consistent with the insertion direction of the starting device 200.

Furthermore, a further improvement is made to the process of the transmission device 300 driving the collection device 400 along the first direction to extend out of the housing 100, specifically as follows:
the transmission device 300 includes a locking assembly 310 and a driving assembly 320; the driving assembly 320 is connected to the collection device 400, and the starting device 200 is detachably connected to the locking assembly 310; the starting device 200 can drive the locking assembly 310 to move, and the locking assembly 310 can restrict the movement of the driving assembly 320 or release the movement restriction on the driving assembly 320; when the locking assembly 310 is locked to the driving assembly 320, the driving assembly 320 cannot drive the collection device 400 to extend; when the starting device 200 is inserted into the locking assembly 310, the locking assembly 310 will move and release the restriction on the movement of the driving assembly 320, and at this time, the driving assembly 320 can drive the collection device 400 to extend and pierce the subject's skin.

Specifically, the starting device 200 drives the locking assembly 310 to slide relative to the housing 100 along a second direction to release the movement restriction on the driving assembly 320; the locking assembly 310 actuates the driving assembly 320, which in turn drives the collection device 400 to move towards outside of the housing 100 along the first direction, which is preferably perpendicular to the second direction.

Furthermore, the locking assembly 310 actuates the driving assembly 320, including:
the locking assembly 310 moves from a first position to a second position along the second direction; a position limiting part 3121B is provided at the end of the locking assembly 310 close to the driving assembly 320; by changing the position relationship between the position limiting part 3121B and the collection device 400, the movement of the collection device 400 can be restricted and unlocked; in the first position, that is, when the starting device 200 is not inserted, the position limiting part 3121B is located at the movement path of the collection device 400 and limits at least part of the collection device 400 within the housing 100, that is, the position limiting part 3121B can at least partially stop the collection device 400 at its movement path; in the second position, that is, after the starting device 200 is inserted, the starting device 200 can drive the locking assembly 310 to move, thereby actuating the position limiting part 3121B to disengage from the driving assembly 320 and releasing the collection device 400.

Furthermore, a feasible solution is provided for the starting device 200 driving the locking assembly 310 to slide along the second direction relative to the housing 100, specifically:
the locking assembly 310 includes a pushing block 311 and sliding pieces 312; the sliding piece 312 can move relative to the pushing block 311, that is, the sliding pieces 312 can move under the action of the pushing block 311; the pushing block 311 is configured to move along the first direction under the action of the starting device 200 and drive the sliding pieces 312, and the sliding pieces 312 are configured to move along the second direction under the action of the pushing block 311.

Specifically, when the starting device 200 is inserted into the locking assembly 310 along the first direction, it will drive the pushing block 311 in the first direction to move synchronously with the starting device 200 in the first direction. At this time, the side walls of the pushing block 311 will squeeze the sliding pieces 312 in the second direction, thereby causing the sliding pieces 312 to move in the second direction and causing the position limiting part 3121B to disengage from the collection device 400, releasing the restriction on the movement of the collection device 400, and then the collection device 400 extends.

Furthermore, a more specific improvement has been made to the following process: the locking assembly 310 actuates the driving assembly 320, which in turn drives the collection device 400 to move towards outside of the housing 100 along the first direction, specifically comprising:
after the collection device 400 disengages from the position limiting part 3121B, the position limiting part 3121B releases the movement restriction on the collection device 400, that is, simultaneously releases the movement of the driving assembly 320, and the collection device 400 extends and retracts by means of the driving action of the driving component. After the restriction on the driving assembly 320 is released, the driving elastic piece 322 in the driving assembly 320 will push the driving shaft 321 associated with the collection device 400 to move, and actuate the collection device 400 to move towards outside of the housing 100 and pierce the skin of the subject.

### Embodiment 30

In this embodiment, a further improvement has been made to the reset of the collection device 400 after piercing the skin.

In this embodiment, the driving assembly 320 further drives the driving shaft 321 and actuates the shaft sleeve 3231 sleeved outside the driving shaft 321 and the collection device 400 to move towards the skin to the blood collection position, thereby compressing the reset elastic piece 3232 sleeved outside the shaft sleeve 3231;
the compressed reset elastic piece 3232 drives the shaft sleeve 321 which actuates the collection device 400 from the blood collection position towards inside of the housing 100 through the driving shaft 321, to move away from the skin of the subject.

When the starting device 200 is inserted into the locking assembly 310, the locking assembly 310 releases the restriction on the movement state of the collection device 400, and it will drives the driving shaft 321, which actuates the shaft sleeve 3231 sleeved outside the driving shaft 321 and the collection device 400 to move towards the skin to the blood collection position. During this process, the reset elastic piece 3232 sleeved outside the shaft sleeve 3231 will be compressed. When the puncture is completed, the compressed reset elastic piece 3232 can drive the shaft sleeve 321 to move towards the original position, and at the same time actuate the driving shaft 321 inside the shaft sleeve 3231 to move synchronously, and the collection device 400 will be actuated by the driving shaft 321 to move from the blood collection position towards inside of the housing 100, to move away from the skin of the subject.

Furthermore, in this embodiment, a collection port 401 is provided on the side of the housing 100 close to the collection device 400, and configured for the collection device 400 to enter and exit. The transmission device 300 drives the collection device 400 along the first direction to extend out of the housing 100 through the collection port 401.

Optionally, the starting device 200 in this embodiment includes a negative pressure tube 210, which has a pressure lower than atmospheric pressure. When the starting device 200 is inserted into the transmission device 300, the negative pressure tube 210 is in communication with the collection port 401 through the second fluid channel 4022.

After the starting device 200 is inserted into the transmission device 300, the negative pressure tube 210 inside the starting device 200 is in communication with the collection port 401, and the skin of the subject is sucked into the collection port 401 by means of the pressure difference between the collection port 401 and the negative pressure tube 210.

The blood or other liquids which are generated after the skin is pierced by the collection device 400 flow into the negative pressure tube 210 through the collection port 401.

Optionally, in other embodiments, the starting device 200 further comprises a connector 220 that is detachably connected to the transmission device 300 and a storage tube 230 extending from the connector 220. The negative pressure tube 210 is in communication with the storage tube 230, and the storage tube 230 is in communication with the second fluid channel 4022 through the connector 220. That is, the blood or other liquids which are generated after the skin is pierced by the collection device 400 flow into the storage tube 230 through the collection port 401 under the negative pressure suction of the negative pressure tube 210.

### Embodiment 31

This embodiment provides a microneedle driving mechanism for driving a microneedle 411A assembly that is extendable and retractable along a first direction, comprising: a housing 100; a driving shaft 321, accommodated inside the housing 100 and connected to the microneedle 411A assembly, wherein the driving shaft 321 is configured to drive the microneedle 411A assembly to extend and retract along the first direction; a driving elastic piece 322, sleeved on the driving shaft 321 and located between an inner wall of the housing 100 and the microneedle 411A assembly, to provide driving force; a position limiting part 3121B, limited at a movement path of the microneedle 411A assembly, wherein the position limiting part 3121B is driven by an external force to be movable relative to the housing 100, that is, by changing the position of the position limiting part 3121B, it can be limited at or disengaged from the movement path of the microneedle 411A assembly; the movement direction of the position limiting part 3121B is not parallel to the first direction. Specifically, when the position limiting part 3121B is limited at the movement path of the microneedle 411A assembly along the first direction, the position limiting part 3121B can limit the movement of the microneedle 411A assembly, and when the position limiting part 3121B is driven by the external force to move relative to the housing 100 along a direction different from the first direction, it can be disengaged from the movement path of the microneedle 411A assembly.

Furthermore, as shown in Figure 3, a further improvement is made to the housing 100, which includes a hollow lower housing 100C supported at the side of the position limiting part 3121B away from the driving shaft 321, and a hollow upper housing 100A fixed relative to the lower housing 100C and limited at the side of the driving shaft 321 away from the lower housing 100C. The configuration of the upper housing 100A and the lower housing 100C not only provides certain support and protection, but also facilitates the layout of other components inside the housing 100, optimizing the layout of the apparatus.

Furthermore, the housing 100 also includes a circular support wall 100B, which is sandwiched and fixed between the upper housing 100A and the lower housing 100C, and surrounds the outer circumferences of the driving shaft 321 and the driving elastic piece 322. The upper housing 100A, the circular support wall 100A, and the lower housing 100C form the housing 100 of the entire apparatus. The overall outer edge of the housing 100 is a circular structure without rigid turning edges, making it convenient for handheld use.

Furthermore, the position limiting part 3121B is sandwiched between the circular support wall 100B and the lower housing 100C along the first direction, and the lower housing 100C is configured to be spaced apart from the position limiting part 3121B along the movement direction of the position limiting part 3121B. There is a gap between the lower housing 100C and the position limiting part 3121B to avoid other needed components.

In other embodiments, the housing 100 further includes a reset mechanism 323 therein, which is located between the driving elastic piece 322 and the circular support wall 100B. The reset mechanism 323 drives the collection device 400 to move towards the upper housing 100A, and the reset mechanism 323 is separated from the driving elastic piece 322.

After the collection is completed, the reset mechanism 323 is activated, which can drive the collection device 400 to move towards the upper housing 100A, so that the microneedle 411A can disengage from the subject's skin and enter the housing 100. During this process, the reset mechanism 323 will separate from the driving elastic piece 322.

Furthermore, the microneedle 411A assembly includes a needle holder 411B and at least one microneedle 411A connected to the needle holder 411B. The driving shaft 321 drives the needle holder 411B to move, thereby actuating the extension and retraction of the microneedle 411A.

Optionally, the needle holder 411B is perforated with a counterbore 411C along the first direction, and the end of the driving shaft 321 is provided with a snapping part which axially extends and can be inserted and fixed in the counterbore 411C to achieve fixation between the needle holder 411B and the end of the driving shaft 321.

As shown in Figure 16, the snapping part includes a snapping body 321B connected to the driving shaft 321, and a shoulder 321C connected to the snapping body 321B, the shoulder 321C can approach or move away from the central axis of the driving shaft 321; the counterbore 411C penetrates the needle holder 411B along the first direction, and the shoulder 321C can pass through the counterbore 411C and abut the side of the needle holder 411B away from the driving shaft 321.

When installing, the shoulder 321C is first passed through the counterbore 411C. The maximum distance of the outer edge of the shoulder 321C is greater than the inner diameter of the counterbore 411C. Since the shoulder 321C can approach or move away from the central axis of the driving shaft 321, when passing through the counterbore 411C, the shoulder 321C can bend inward under the pressure from the side wall of the counterbore 411C, and then its outer edge can be inserted into the counterbore 411C. When the shoulder 321C passes through the needle holder 411B, the side wall of the counterbore 411C no longer squeezes the outer edge of the shoulder 321C, and the shoulder 321C will return to its original state and abut the side of the needle holder 411B away from the driving shaft 321. At this time, the snapping body 321B is fixed inside the counterbore 411C.

Optionally, the snapping part includes a pair of snapping bodies 321B connected to the driving shaft 321. The two snapping bodies 321B are arranged opposite each other and each has a shoulder 321C. The shoulder 321C is an inverted right angled triangle, and the side of the shoulder 321C that abuts the side wall of the counterbore 411C is the oblique edge, making it easy to insert shoulder 321C into the counterbore 411C.

Optionally, the side of the needle holder 411B away from the driving shaft 321 is provided with a snapping groove 411D that can accommodate the snapping shoulder. The snapping groove 411D is recessed into the needle holder 411B, and when the snapping body 321B passes through the counterbore 411C, the shoulder 321C can be snapped into the snapping groove 411D.

Although the embodiments of this application have been shown and described above, it can be understood that the above embodiments are exemplary and should not be construed as limiting this application. Ordinary skilled in the art can make changes, modifications, substitutions, and variations to the above embodiments within the scope of this application.

## Claims

1. An apparatus for collecting blood or other liquids, **characterized in that**, the apparatus for collecting blood or other liquids comprises a housing, a starting device, a transmission device and a collection device;
the collection device is housed in the housing;
the transmission device is housed in the housing, the transmission device comprises a locking assembly and a driving assembly, the driving assembly is connected to the collection device, at least part of the starting device is detachably connected to the locking assembly, and the locking assembly is configured to restrict a movement of the driving assembly before the starting device is inserted, and to release a movement restriction on the driving assembly after the starting device is inserted, so that the driving assembly actuates the collection device to extend out of the housing.

2. The apparatus for collecting blood or other liquids according to claim 1, **characterized in that**, the locking assembly comprises a pushing block and sliding pieces, the pushing block is movably connected to the sliding pieces, the pushing block is configured to move along a first direction under an action of the starting device and drive the sliding pieces, and the sliding pieces are configured to move along a second direction under an action of the pushing block, for restricting a movement of the collection device in the first direction and releasing a movement restriction on the collection device.

3. The apparatus for collecting blood or other liquids according to claim 2, **characterized in that**, the sliding piece comprises a first slider and a first deformable piece located between the first slider and an inner wall of the housing, the first deformable piece is connected to the first slider for reciprocating the first slider along the second direction.

4. The apparatus for collecting blood or other liquids according to claim 2, **characterized in that**, the sliding pieces are symmetrically arranged on both sides of the collection device along the second direction, and the pushing block is arranged between the two sliding pieces and pushes the two sliding pieces to move in opposite directions.

5. The apparatus for collecting blood or other liquids according to claim 3, **characterized in that**, the first slider has a body part and a position limiting part, the body part is connected to the first deformable piece, the position limiting part protrudes from the body part along the second direction, and the position limiting part is configured to at least partially stop the collection device in the first direction after the starting device is removed, and release a stop on the collection device after the starting device is inserted.

6. The apparatus for collecting blood or other liquids according to claim 2, **characterized in that**, the pushing block has an inserting-connection part and squeezing parts, the inserting-connection part is configured to form an inserting connection with the starting device, and the squeezing parts protrude from an outer side of the inserting-connection part for squeezing the sliding pieces in the second direction.

7. The apparatus for collecting blood or other liquids according to claim 6, **characterized in that**, a cross-sectional area of the squeezing part gradually decreases along an insertion direction of the starting device.

8. The apparatus for collecting blood or other liquids according to claim 6, **characterized in that**,
the housing is provided with a guiding column;
the inserting-connection part comprises an inserting-connection section and a guiding section, the inserting-connection section is provided with an inserting-connection groove, the guiding section is sleeved on the guiding column, and the squeezing part is provided on the guiding section.

9. The apparatus for collecting blood or other liquids according to claim 6, **characterized in that**, the sliding piece is provided with a positioning portion corresponding to the squeezing parts, and the positioning portion is provided with accommodation parts that can accommodate the squeezing parts.

10. The apparatus for collecting blood or other liquids according to claim 2, **characterized in that**, the driving assembly is located between the collection device and an inner wall of the housing, the driving assembly comprises a driving shaft connected to the collection device and a driving elastic piece sleeved outside the driving shaft, and after the sliding pieces release the movement restriction on the collection device, the driving elastic piece pushes the driving shaft along the first direction and actuates the collection device to move towards outside of the housing to a collection position.

11. The apparatus for collecting blood or other liquids according to claim 10, **characterized in that**, the driving assembly further comprises a reset mechanism, which is configured to push the collection device to move towards inside of the housing after the collection device moves to the collection position, so as to reset the collection device.

12. The apparatus for collecting blood or other liquids according to claim 11, **characterized in that**, the reset mechanism comprises a shaft sleeve sleeved outside the driving shaft and a reset elastic piece connected to the shaft sleeve, and the driving shaft is configured to pass through the shaft sleeve along the first direction and has a protrusion protruding outward; after the collection device moves to the collection position, the reset elastic piece pushes the shaft sleeve and actuates the driving shaft and the collection device to move towards inside of the housing through the protrusion.

13. The apparatus for collecting blood or other liquids according to claim 12, **characterized in that**, an end of the reset elastic piece away from the collection device is fixed to the shaft sleeve, and an end of the reset elastic piece close to the collection device is configured to be movable relative to the shaft sleeve along the first direction relative to the housing.

14. The apparatus for collecting blood or other liquids according to claim 12, **characterized in that**, both the driving elastic piece and the reset elastic piece are precompressed springs, the reset elastic piece is sleeved on an outer circumference of the shaft sleeve, and an elastic coefficient of the driving elastic piece is greater than that of the reset elastic piece.

15. The apparatus for collecting blood or other liquids according to claim 12, **characterized in that**, the driving assembly further comprises a top position-limiting mechanism located at an end of the driving elastic piece away from the collection device, and the top position-limiting mechanism is configured to precompress the driving elastic piece and the reset elastic piece.

16. The apparatus for collecting blood or other liquids according to claim 15, **characterized in that**, the top position-limiting mechanism comprises second sliders located on opposite sides of the driving shaft and position-limiting elastic pieces for actuating two second sliders to move relative to each other, at least two second sliders enclose an unobstructed part, and an end of the driving shaft away from the collection device extends outside the top position-limiting mechanism through the unobstructed part and is spaced apart from the top position-limiting mechanism.

17. The apparatus for collecting blood or other liquids according to claim 16, **characterized in that**, the position-limiting elastic piece is a precompressed position-limiting elastic piece connected between the two second sliders which are arranged opposite to each other, and the two second sliders and the precompressed position-limiting elastic pieces enclose the unobstructed part; or
the position-limiting elastic piece is a pre-stretched position-limiting elastic piece connected between the slider and the housing, and the two second sliders are spaced to form the unobstructed part therebetween.

18. The apparatus for collecting blood or other liquids according to claim 16, **characterized in that**, a position-limiting groove is provided on a side of the second slider close to the collection device, and the position-limiting groove is configured to form a snapping connection with the shaft sleeve;
when the collection device moves to the collection position, the driving elastic piece drives the driving shaft to actuate the shaft sleeve to move and disengage from the position-limiting groove, the position-limiting elastic piece drives the two second sliders to separate, the end of the driving elastic piece away from the collection device is released through a gap generated by a separation between the second sliders, and the reset elastic piece pushes the shaft sleeve and actuates the driving shaft and the collection device to move towards inside of the housing through the protrusion.

19. The apparatus for collecting blood or other liquids according to any one of claims 2-18, **characterized in that**, the first direction is perpendicular to the second direction.

20. The apparatus for collecting blood or other liquids according to claim 19, **characterized in that**, the housing is provided with an installation port and a collection port, the starting device is detachably connected to the locking assembly via the installation port, and the transmission device drives the collection device to extend out of the housing through the collection port along the first direction.

21. The apparatus for collecting blood or other liquids according to claim 20, **characterized in that**, a first fluid channel which is in communication with the collection device is housed in the housing, and the starting device is in communication with the collection device through the first fluid channel.

22. The apparatus for collecting blood or other liquids according to claim 20, **characterized in that**, the starting device comprises a starting assembly and a first puncturing assembly, the starting assembly is detachably connected to the locking assembly, the first puncturing assembly is detachably connected to the starting assembly, and the first puncturing assembly is configured to pierce the starting assembly when the starting assembly is inserted into the locking assembly of the transmission device, so that the starting assembly is in communication with the collection device via the first puncturing assembly.

23. The apparatus for collecting blood or other liquids according to claim 22, **characterized in that**, the starting assembly comprises a connector, a storage tube extending from the connector, and a negative pressure tube in communication with the storage tube, the connector is configured to be detachably connected to the locking assembly, the negative pressure tube has a pressure lower than atmospheric pressure, and the storage tube is configured to be in communication with the collection device through the first puncturing assembly when the connector is inserted into the locking assembly, for storing the blood or other liquids collected by the collection device.

24. The apparatus for collecting blood or other liquids according to claim 23, **characterized in that**, one of the first puncturing assembly and the starting assembly comprises a first piercing piece which has a hollow tubular shape with two end installation ports, and the other one comprises a first puncturable seal corresponding to the first piercing piece, and the first piercing piece is configured to pierce the first puncturable seal when the connector is inserted into the locking assembly, to establish communication between the storage tube and the collection device.

25. The apparatus for collecting blood or other liquids according to claim 24, **characterized in that**, the starting assembly further comprises a capillary tube connected to the storage tube, and the storage tube is in communication with the negative pressure tube through the capillary tube.

26. The apparatus for collecting blood or other liquids according to claim 25, **characterized in that**, the storage tube comprises a first open end connected to the connector, and a second open end opposite to the first open end, a first seal is fitted inside the second open end, and the capillary tube penetrates and is fixed to the first seal and extends into the storage tube through the second open end.

27. The apparatus for collecting blood or other liquids according to claim 26, **characterized in that**, the negative pressure tube is coaxially sleeved outside of the storage tube, and the starting assembly further comprises a second seal sandwiched between an outer wall of the storage tube and an inner wall of the negative pressure tube.

28. The apparatus for collecting blood or other liquids according to claim 27, **characterized in that**, a positioning part protruding from the outer wall of the storage tube is provided, an end of the negative pressure tube close to the connector abuts a side of the positioning part away from the first open end, and part of the second seal is sandwiched between the positioning part and the negative pressure tube.

29. The apparatus for collecting blood or other liquids according to claim 28, **characterized in that**, the starting assembly comprises a first puncturable seal that seals the first open end, the first puncturable seal comprises a fixed part which is located outside the installation port end and has a size larger than a diameter of the storage tube; and a punctured part extending from the fixed part into the installation port end and matching the diameter of the storage tube.

30. The apparatus for collecting blood or other liquids according to claim 29, **characterized in that**, the connector comprises a crown cover that is sleeved on an outer circumference of the fixed part and extends to an outer circumference of the storage tube.

31. The apparatus for collecting blood or other liquids according to claim 29, **characterized in that**, the fixed part is provided with an unobstructed recess corresponding to the first puncturable seal and extending to the punctured part, and when the connector is inserted into the locking assembly, the first piercing piece pierces the punctured part through the unobstructed recess and is in communication with the storage tube.

32. The apparatus for collecting blood or other liquids according to claim 1, **characterized in that**, the collection device comprises a second puncturing assembly, a second puncturable seal, and a blood collection assembly, at least part of the blood collection assembly is provided at the collection port and located inside the housing, the second puncturable seal is provided between the housing and the blood collection assembly, and the second puncturing assembly is configured to extend out of the housing through the second puncturable seal under an action of the driving assembly.

33. The apparatus for collecting blood or other liquids according to claim 32, **characterized in that**, the blood collection assembly comprises a first base plate and a second base plate which are stacked along a first direction, the first base plate and the second base plate are arranged outside the collection port and connected to the housing, the first base plate and the second base plate collectively form a collection chamber, and the collection chamber is configured to have an internal pressure lower than atmospheric pressure when the starting device is inserted into the locking assembly, to collect blood or other liquids.

34. The apparatus for collecting blood or other liquids according to claim 33, **characterized in that**, the second puncturable seal comprises a first sealing part, an elastic deformation part, and a second sealing part, the elastic deformation part is located between the first sealing part and the second sealing part, the first sealing part is configured to be pierced by the second puncturable seal, and the second sealing part is provided at the collection port.

35. The apparatus for collecting blood or other liquids according to claim 34, **characterized in that**, the elastic deformation part is formed as a hollow frustum-shaped structure in an initial state.

36. The apparatus for collecting blood or other liquids according to claim 34, **characterized in that**, the second puncturing assembly comprises a needle holder and at least one microneedle fixed to the needle holder, and the first base plate is provided with a through hole corresponding to the at least one microneedle one by one.

37. The apparatus for collecting blood or other liquids according to claim 36, **characterized in that**, the first base plate is further provided with a second fluid channel, which is in communication with the collection chamber.

38. The apparatus for collecting blood or other liquids according to claim 37, **characterized in that**, at least part of the fluid channel is formed as a wavy structure.

39. The apparatus for collecting blood or other liquids according to claim 38, **characterized in that**, the second base plate is provided with a penetrated hole, the first base plate is provided with a central part protruding towards the penetrated hole, and the central part is provided in the penetrated hole and a gap is formed between the central part and the penetrated hole;
the through hole is provided at a position corresponding to the gap.

40. The apparatus for collecting blood or other liquids according to claim 39, **characterized in that**, a surface of the central part away from the first base plate is flush with a surface of the second base plate away from the first base plate.

41. The apparatus for collecting blood or other liquids according to claim 40, **characterized in that**, an adhesive layer is further provided on a peripheral side of the second base plate, for adhering the surface of the second base plate away from the first base plate to a skin.

42. An apparatus for collecting blood or other liquids, **characterized in that**, the apparatus for collecting blood or other liquids comprises a housing, a starting device, a transmission device and a collection device;
the housing is provided with an installation port and a collection port;
the transmission device is housed in the housing, and at least part of the starting device is housed in the housing and connected to the transmission device through the installation port;
at least part of the collection device is housed in the housing, and the collection device collects blood or other liquids through the collection port;
the collection port and the installation port are not coaxially arranged.

43. The apparatus for collecting blood or other liquids according to claim 42, **characterized in that**, the housing comprises an upper housing and a lower housing, the lower housing is perforated with the collection port through which the collection device is extended, and the upper housing is perforated with the installation port for inserting the starting device.

44. The apparatus for collecting blood or other liquids according to claim 43, **characterized in that**, a projection of the collection port onto the upper housing along an extension direction of the collection device does not overlap with the installation port.

45. The apparatus for collecting blood or other liquids according to claim 44, **characterized in that**, the transmission device comprises a locking assembly and a driving assembly, the driving assembly is connected to the collection device, at least part of the starting device is detachably connected to the locking assembly, and the locking assembly is configured to release a movement restriction on the driving assembly after the starting device is inserted, so that the driving assembly actuates the collection device to extend out of the housing, and the locking assembly is configured to restrict a movement of the driving assembly after the starting device is removed.

46. The apparatus for collecting blood or other liquids according to claim 45, **characterized in that**, the locking assembly comprises a pushing block and sliding pieces, the pushing block is movably connected to the sliding pieces, the pushing block is configured to move along a first direction under an action of the starting device and drive the sliding pieces, and the sliding pieces are configured to move along a second direction under an action of the pushing block, for restricting a movement of the collection device in the first direction and releasing a movement restriction on the collection device.

47. The apparatus for collecting blood or other liquids according to claim 46, **characterized in that**, the sliding piece comprises a first slider and a first deformable piece located between the first slider and an inner wall of the housing, the first deformable piece is connected to the first slider for reciprocating the first slider along the second direction.

48. The apparatus for collecting blood or other liquids according to claim 46, **characterized in that**, the sliding pieces are symmetrically arranged on both sides of the collection device along the second direction, and the pushing block is arranged between the two sliding pieces and pushes the two sliding pieces to move in opposite directions.

49. The apparatus for collecting blood or other liquids according to claim 47, **characterized in that**, the first slider has a body part and a position limiting part, the body part is connected to the first deformable piece, the position limiting part protrudes from the body part along the second direction, and the position limiting part is configured to at least partially stop the collection device in the first direction after the starting device is removed, and release a stop on the collection device after the starting device is inserted.

50. The apparatus for collecting blood or other liquids according to claim 46, **characterized in that**, the pushing block has an inserting-connection part and squeezing parts, the inserting-connection part is configured to form an inserting connection with the starting device, and the squeezing parts protrude from an outer side of the inserting-connection part for squeezing the sliding pieces in the second direction.

51. The apparatus for collecting blood or other liquids according to claim 50, **characterized in that**, a cross-sectional area of the squeezing part gradually decreases along an insertion direction of the starting device.

52. The apparatus for collecting blood or other liquids according to claim 50, **characterized in that**,
the housing is provided with a guiding column;
the inserting-connection part comprises an inserting-connection section and a guiding section, the inserting-connection section is provided with an inserting-connection groove, the guiding section is sleeved on the guiding column, and the squeezing part is provided on the guiding section.

53. The apparatus for collecting blood or other liquids according to claim 50, **characterized in that**, the sliding piece is provided with a positioning portion corresponding to the squeezing parts, and the positioning portion is provided with accommodation parts that can accommodate the squeezing parts.

54. The apparatus for collecting blood or other liquids according to claim 46, **characterized in that**, the driving assembly is located between the collection device and an inner wall of the housing, the driving assembly comprises a driving shaft connected to the collection device and a precompressed driving elastic piece sleeved outside the driving shaft, and after the sliding pieces release the movement restriction on the collection device, the precompressed driving elastic piece pushes the driving shaft along the first direction and actuates the collection device to move towards outside of the housing to a collection position.

55. The apparatus for collecting blood or other liquids according to claim 54, **characterized in that**, the driving assembly further comprises a reset mechanism, which is configured to push the collection device to move towards inside of the housing after the collection device moves to the collection position, so as to reset the collection device.

56. The apparatus for collecting blood or other liquids according to claim 55, **characterized in that**, the reset mechanism comprises a shaft sleeve sleeved outside the driving shaft and a reset elastic piece connected to the shaft sleeve, and the driving shaft is configured to pass through the shaft sleeve along the second direction and has a protrusion protruding outward; after the collection device moves to the collection position, the reset elastic piece pushes the shaft sleeve and actuates the driving shaft and the collection device to move towards inside of the housing through the protrusion.

57. The apparatus for collecting blood or other liquids according to claim 56, **characterized in that**, an end of the reset elastic piece away from the collection device is fixed to the shaft sleeve, and an end of the reset elastic piece close to the collection device is configured to be movable relative to the shaft sleeve along the second direction relative to the housing.

58. The apparatus for collecting blood or other liquids according to claim 57, **characterized in that**, both the driving elastic piece and the reset elastic piece are precompressed springs, the reset elastic piece is sleeved on an outer circumference of the shaft sleeve, and an elastic coefficient of the driving elastic piece is greater than that of the reset elastic piece.

59. The apparatus for collecting blood or other liquids according to claim 57, **characterized in that**, the driving assembly further comprises a top position-limiting mechanism located at an end of the precompressed driving elastic piece away from the collection device, and the top position-limiting mechanism is configured to precompress the precompressed driving elastic piece and the reset elastic piece.

60. The apparatus for collecting blood or other liquids according to claim 59, **characterized in that**, the top position-limiting mechanism comprises second sliders located on opposite sides of the driving shaft and position-limiting elastic pieces for actuating two second sliders to move relative to each other, at least two second sliders enclose an unobstructed part, and an end of the driving shaft away from the collection device extends outside the top position-limiting mechanism through the unobstructed part and is spaced apart from the top position-limiting mechanism.

61. The apparatus for collecting blood or other liquids according to claim 60, **characterized in that**, the position-limiting elastic piece is a precompressed position-limiting elastic piece connected between the two second sliders which are arranged opposite to each other, and the two second sliders and the precompressed position-limiting elastic pieces enclose the unobstructed part; or
the position-limiting elastic piece is a pre-stretched position-limiting elastic piece connected between the slider and the housing, and the two second sliders are spaced to form the unobstructed part therebetween.

62. The apparatus for collecting blood or other liquids according to claim 60, **characterized in that**, a position-limiting groove is provided on a side of the second slider close to the collection device, and the position-limiting groove is configured to form a snapping connection with the shaft sleeve;
when the collection device moves to the collection position, the driving elastic piece drives the driving shaft to actuate the shaft sleeve to move and disengage from the position-limiting groove, the position-limiting elastic piece drives the two second sliders to separate, an end of the driving elastic piece away from the collection device is released through a gap generated by a separation between the second sliders, and the reset elastic piece pushes the shaft sleeve and actuates the driving shaft and the collection device to move towards inside of the housing through the protrusion.

63. The apparatus for collecting blood or other liquids according to any one of claims 46-62, **characterized in that**, the first direction is perpendicular to the second direction.

64. The apparatus for collecting blood or other liquids according to claim 63, **characterized in that**, a first fluid channel is provided inside the housing, and the starting device is in communication with the collection device through the first fluid channel.

65. The apparatus for collecting blood or other liquids according to claim 63, **characterized in that**, the starting device comprises a starting assembly and a first puncturing assembly, the starting assembly is detachably connected to the locking assembly, the first puncturing assembly is detachably connected to the starting assembly, and the first puncturing assembly is configured to pierce the starting assembly when the starting assembly is inserted into the locking assembly of the transmission device, so that the starting assembly is in communication with the collection device via the first puncturing assembly.

66. The apparatus for collecting blood or other liquids according to claim 65, **characterized in that**, the starting assembly comprises a connector, a storage tube extending from the connector, and a negative pressure tube in communication with the storage tube, the connector is configured to be detachably connected to the locking assembly, the negative pressure tube has a pressure lower than atmospheric pressure, and the storage tube is configured to be in communication with the collection device through the first puncturing assembly when the connector is inserted into the locking assembly, for storing the blood or other liquids collected by the collection device.

67. The apparatus for collecting blood or other liquids according to claim 66, **characterized in that**, one of the first puncturing assembly and the starting assembly comprises a first piercing piece which has a hollow tubular shape with two open ends, and the other one comprises a first puncturable seal corresponding to the first piercing piece, and the first piercing piece is configured to pierce the first puncturable seal when the connector is inserted into the locking assembly, to establish communication between the storage tube and the collection device.

68. The apparatus for collecting blood or other liquids according to claim 67, **characterized in that**, the starting assembly further comprises a capillary tube connected to the storage tube, and the storage tube is in communication with the negative pressure tube through the capillary tube.

69. The apparatus for collecting blood or other liquids according to claim 68, **characterized in that**, the storage tube comprises a first open end connected to the connector, and a second open end opposite to the first open end, a first seal is fitted inside the second open end, and the capillary tube penetrates and is fixed to the first seal and extends into the storage tube through the second open end.

70. The apparatus for collecting blood or other liquids according to claim 69, **characterized in that**, the negative pressure tube is coaxially sleeved outside of the storage tube, and the starting assembly further comprises a second seal sandwiched between an outer wall of the storage tube and an inner wall of the negative pressure tube.

71. The apparatus for collecting blood or other liquids according to claim 70, **characterized in that**, a positioning part protruding from the outer wall of the storage tube is provided, an end of the negative pressure tube close to the connector abuts a side of the positioning part away from the first open end, and part of the second seal is sandwiched between the positioning part and the negative pressure tube.

72. The apparatus for collecting blood or other liquids according to claim 71, **characterized in that**, the starting assembly comprises a first puncturable seal that seals the first open end, the first puncturable seal comprises a fixed part which is located outside the open end and has a size larger than a diameter of the storage tube; and a punctured part extending from the fixed part into the open end and matching the diameter of the storage tube.

73. The apparatus for collecting blood or other liquids according to claim 72, **characterized in that**, the connector comprises a crown cover that is sleeved on an outer circumference of the fixed part and extends to an outer circumference of the storage tube.

74. The apparatus for collecting blood or other liquids according to claim 72, **characterized in that**, the fixed part is provided with an unobstructed recess corresponding to the first puncturable seal and extending to the punctured part, and when the connector is inserted into the locking assembly, the first piercing piece pierces the punctured part through the unobstructed recess and is in communication with the storage tube.

75. The apparatus for collecting blood or other liquids according to claim 46, **characterized in that**, the collection device comprises a second puncturing assembly and a blood collection assembly, at least part of the blood collection assembly is provided at the collection port and located inside the housing, and the second puncturing assembly is configured to extend out of the housing after piercing the blood collection assembly under an action of the driving assembly.

76. The apparatus for collecting blood or other liquids according to claim 75, **characterized in that**, the blood collection assembly comprises a second puncturable seal, a first base plate and a second base plate which are stacked along the first direction, the second puncturable seal is provided at the collection port and located inside the housing, the first base plate and the second base plate are arranged outside the collection port and connected to the housing, the second puncturable seal and the first, second base plates are enclosed to form a collection chamber, and the collection chamber is configured to have an internal pressure lower than atmospheric pressure when the starting device is inserted into the locking assembly, to collect blood or other liquids.

77. The apparatus for collecting blood or other liquids according to claim 76, **characterized in that**, the second puncturable seal comprises a first sealing part, an elastic deformation part, and a second sealing part, the elastic deformation part is located between the first sealing part and the second sealing part, the first sealing part is configured to be pierced by the second puncturable seal, and the second sealing part is provided at the collection port.

78. The apparatus for collecting blood or other liquids according to claim 77, **characterized in that**, the elastic deformation part is formed as a hollow frustum-shaped structure in an initial state.

79. The apparatus for collecting blood or other liquids according to claim 78, **characterized in that**, the first base plate is further provided with a second fluid channel, which is in communication with the collection chamber.

80. The apparatus for collecting blood or other liquids according to claim 79, **characterized in that**, at least part of the fluid channel is formed as a wavy structure.

81. The apparatus for collecting blood or other liquids according to claim 79, **characterized in that**, the second base plate is provided with a penetrated hole, the first base plate is provided with a central part protruding towards the penetrated hole, and the central part is provided in the penetrated hole and a gap is formed between the central part and an inner wall of the penetrated hole;
the second puncturing assembly is provided at a position corresponding to the gap.

82. The apparatus for collecting blood or other liquids according to claim 81, **characterized in that**, a surface of the central part away from the first base plate is flush with a surface of the second base plate away from the first base plate.

83. A method for collecting blood or other liquids from a subject, **characterized in that**, the method for collecting blood or other liquids from a subject comprises:
applying a housing to a skin of the subject, wherein the housing is provided with a collection device therein, which is extendable and retractable relative to the housing, and a transmission device associated with the collection device; the transmission device comprises a locking assembly and a driving assembly, wherein the driving assembly is connected to the collection device;
inserting a starting device into the transmission device, the starting device driving the locking assembly to slide relative to the housing along a second direction, to release a movement restriction on the driving assembly;
enabling the locking assembly to actuate the driving assembly, which drives the collection device to move along a first direction towards outside of the housing to pierce the skin of the subject.

84. The method for collecting blood or other liquids from a subject according to 83, **characterized in that**, enabling the locking assembly to actuate the driving assembly, comprises:
moving the locking assembly from a first position to a second position along the second direction, an end of the locking assembly close to the driving assembly being provided with a position limiting part; in the first position, the position limiting part being located at a movement path of the collection device and limiting at least part of the collection device within the housing; in the second position, the position limiting part disengaging from the driving assembly and releasing the collection device.

85. The method for collecting blood or other liquids from a subject according to claim 83, **characterized in that**, the starting device driving the locking assembly to slide relative to the housing along the second direction, comprises:
the locking assembly comprising a pushing block and sliding pieces, wherein the pushing block is configured to move along the first direction under an action of the starting device and drive the sliding pieces, and the sliding pieces are configured to move in the second direction under an action of the pushing block.

86. The method for collecting blood or other liquids from a subject according to claim 84, **characterized in that**, the locking assembly actuating the driving assembly, which drives the collection device to move along the first direction towards outside of the housing, comprises:
after the collection device disengaging from the position limiting part, a driving elastic piece in the driving assembly pushing a driving shaft associated with the collection device, and actuating the collection device to move towards outside of the housing and pierce the skin of the subject.

87. The method for collecting blood or other liquids from a subject according to claim 86, **characterized in that**, the method further comprises:
enabling the driving assembly to further drive the driving shaft and actuate a shaft sleeve sleeved outside the driving shaft and the collection device to move towards the skin to a blood collection position, thereby compressing a reset elastic piece sleeved outside the shaft sleeve;
enabling the compressed reset elastic piece to drive the shaft sleeve, which actuate the collection device to move from the blood collection position towards inside of the housing through the driving shaft, to move away from the skin of the subject.

88. The method for collecting blood or other liquids from a subject according to claim 87, **characterized in that**, a collection port is provided on a side of the housing close to the collection device, and the transmission device drives the collection device to extend out of the housing through the collection port along the first direction, and the method further comprises:
after the starting device is inserted into the transmission device, enabling a negative pressure tube inside the starting device to be in communication with the collection port, the skin of the subject being sucked into the collection port by means of a pressure difference between the collection port and the negative pressure tube;
enabling the blood or other liquids which are generated after the skin is pierced by the collection device to flow into the negative pressure tube through the collection port.

89. A microneedle driving mechanism for driving a microneedle assembly which is extendable and retractable along a first direction, **characterized in that**, the microneedle driving mechanism comprises:
a housing;
a driving shaft, accommodated within the housing and connected to the microneedle assembly;
a driving elastic piece, sleeved on the driving shaft, wherein the driving elastic piece is located between an inner wall of the housing and the microneedle assembly;
a position limiting part, limited at a movement path of the microneedle assembly, wherein the position limiting part is driven by an external force to be movable relative to the housing;
wherein a movement direction of the position limiting part is not parallel to the first direction.

90. The microneedle driving mechanism according to claim 89, **characterized in that**, the housing comprises a hollow lower housing supported at a side of the position limiting part away from the driving shaft, and a hollow upper housing fixed relative to the lower housing and limited at a side of the driving shaft away from the lower housing.

91. The microneedle driving mechanism according to claim 90, **characterized in that**, the housing further comprises a circular support wall, which is sandwiched and fixed between the upper housing and the lower housing, and surrounds outer circumferences of the driving shaft and the driving elastic piece.

92. The microneedle driving mechanism according to claim 91, **characterized in that**, the position limiting part is sandwiched between the circular support wall and the lower housing along the first direction, and the lower housing is configured to be spaced apart from the position limiting part along a movement direction of the position limiting part.

93. The microneedle driving mechanism according to claim 91, **characterized in that**, the microneedle driving mechanism further comprises a reset mechanism located between the driving elastic piece and the circular support wall, wherein the reset mechanism drives the microneedle assembly to move towards the upper housing, and the reset mechanism is separated from the driving elastic piece.

94. The microneedle driving mechanism according to any one of claims 89-93, **characterized in that**, the microneedle assembly comprises a needle holder and at least one microneedle connected to the needle holder.

95. The microneedle driving mechanism according to claim 94, **characterized in that**, the needle holder is perforated with a counterbore along the first direction, and an end of the driving shaft is provided with a snapping part which axially extends and can be inserted and fixed in the counterbore.

96. The microneedle driving mechanism according to claim 95, **characterized in that**, the snapping part comprises a snapping body connected to the driving shaft, and a shoulder connected to the snapping body, wherein the shoulder can approach or move away from a central axis of the driving shaft;
the counterbore penetrates the needle holder along the first direction, and the shoulder can pass through the counterbore and abut a side of the needle holder away from the driving shaft.

97. The microneedle driving mechanism according to claim 96, **characterized in that**, the side of the needle holder away from the driving shaft is provided with a snapping groove that can accommodate the shoulder.
